(19) Europäisches Patentamt
European Patent Office
Office européen des brevets

(11) Veröffentlichungsnummer: **0 434 624 B1**

(12) **EUROPÄISCHE PATENTSCHRIFT**

(45) Veröffentlichungstag der Patentschrift: **08.02.95**

(51) Int. Cl.[6]: **C07D 487/04**, A01N 43/90, //(C07D487/04,249:00,239:00)

(21) Anmeldenummer: **90810986.1**

(22) Anmeldetag: **13.12.90**

Die Akte enthält technische Angaben, die nach dem Eingang der Anmeldung eingereicht wurden und die nicht in dieser Patentschrift enthalten sind.

# (54) Triazolylsulfonamide.

(30) Priorität: **22.12.89 CH 4643/89**
**14.08.90 CH 2638/90**

(43) Veröffentlichungstag der Anmeldung:
**26.06.91 Patentblatt 91/26**

(45) Bekanntmachung des Hinweises auf die Patenterteilung:
**08.02.95 Patentblatt 95/06**

(84) Benannte Vertragsstaaten:
**CH DE FR GB LI**

(56) Entgegenhaltungen:
**EP-A- 0 142 152**
**EP-A- 0 375 076**
**EP-A- 0 378 508**
**DD-A- 70 311**

(73) Patentinhaber: **CIBA-GEIGY AG**
**Klybeckstrasse 141**
**CH-4002 Basel (CH)**

(72) Erfinder: **Dürr, Dieter, Dr.**
**Brändelistalweg 16**
**CH-4103 Bottmingen (CH)**

EP 0 434 624 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft herbizid wirksame und pflanzenwuchsregulierende N-Phenyl-triazolopyrimidinylsulfonamide, Verfahren zu ihrer Herstellung, die sie als Wirkstoffe enthaltende Mittel sowie deren Verwendung zum Bekämpfen von Unkräutern, vor allem selektiv in Nutzpflanzenkulturen oder zum Regulieren und Hemmen des Pflanzenwachstums.

Triazolylsulfonamide mit herbizider Wirkung sind aus der Europäischen Patentanmeldung Nr. 0 142 152 bekannt. Die dort offenbarten Wirkstoffe können jedoch die Anforderungen bezüglich Wirkungsstärke, Selektivität und Persistenz nicht immer erfüllen. Es besteht somit ein Bedarf nach besser wirksamen und selektiveren Wirkstoffen.

Es wurden nun neue Triazolylsulfonamide mit verbesserter herbizider und pflanzenwuchsregulierender Wirkung gefunden.

Die erfindungsgemässen N-Phenyl-triazolopynmidinylsulfonamide entsprechen der Formel I

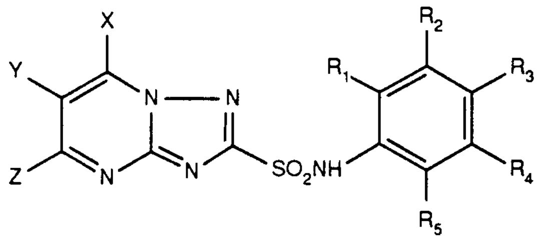

(I)

worin

$R_1$ für Halogen, Phenyl, O-Phenyl, $C_1$-$C_4$-Alkyl, $C_1$-$C_4$-Halogenalkyl, $-OR_8$, $-OR_9$, Nitro, Hydroxy, Cyano, $-SR_6$, $-SOR_6$, $-SO_2R_6$, $-SR_8$, $-SOR_8$, $-SO_2R_8$, $-SO_3R_8$, $-SR_9$, $SOR_9$, $-SO_2R_9$, $-SO_2NR_{10}(R_{10})$, $-SO_3R_9$, $-COOR_7$, $-CONHR_9$, $-CONR_9(R_9)$, Formyl,

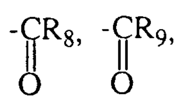

$-NH_2$, $NHR_9$ oder $-NR_9(R_9)$;

$R_2$ und $R_4$ unabhängig voneinander für Wasserstoff, Halogen, $C_1$-$C_4$-Alkyl, $-COOR_7$, oder $-OR_9$;

$R_3$ für Wasserstoff oder Halogen;

$R_5$ für Wasserstoff, Halogen, Phenyl, O-Phenyl, $C_1$-$C_4$-Alkyl, $C_1$-$C_4$-Halogenalkyl, $-OR_8$, $-OR_9$, Nitro, Hydroxy, Cyano, $SR_6$, $-SOR_6$, $-SO_2R_6$, $-SR_8$, $-SOR_8$, $-SO_3R_8$, $-SR_9$, $SOR_9$, $-SO_2R_9$, $-SO_2NR_{10}(R_{10})$, $-SO_3R_9$, $-COOR_7$, $-CONHR_9$, $-CONR_9(R_9)$, Formyl,

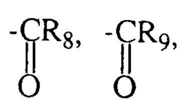

$-NH_2$, $NHR_9$ oder $-NR_9(R_9)$;

$R_6$ für Wasserstoff, Phenyl, Benzyl, $C_2$-$C_4$-Alkenyl, $C_2$-$C_4$-Alkinyl oder durch $-OR_9$ substituiertes $C_1$-$C_4$-Alkyl;

$R_7$ für $C_1$-$C_4$-Alkyl, $C_2$-$C_4$-Alkenyl, $C_2$-$C_4$-Alkinyl, Phenyl oder Benzyl;

$R_8$ für $C_1$-$C_4$-Halogenalkyl;

$R_9$ für $C_1$-$C_4$-Alkyl;

$R_{10}$ für Wasserstoff oder $C_1$-$C_4$-Alkyl;

X, Y, und Z unabhängig voneinander für Wasserstoff, $C_1$-$C_4$-Alkyl, $C_1$-$C_4$-Halogenalkyl, Phenylthio, Benzylthio, Hydroxy, Halogen, $-OR_8$, $-OR_9$, durch $-OR_9$ substituiertes $C_1$-$C_4$-Alkyl, durch $-OR_8$ substituiertes $C_1$-$C_4$-Alkyl, $-NH_2$, $-NHR_9$, $-NR_9(R_9)$, $C_3$-$C_6$-Cycloalkyl, durch Halogen, $-OR_8$, $-OR_9$, $-SR_8$, $-SR_9$, oder $C_1$-$C_4$-Alkyl substituiertes $C_3$-$C_6$-Cycloalkyl oder X und Y zusammen oder Y und Z zusammen bilden eine $C_2$-$C_3$-Alkylenbrücke, die durch Sauerstoff oder Schwefel unterbrochen sein kann; stehen,

mit der Massgabe, dass mindestens einer der Substituenten X, Y und Z für $C_3$-$C_6$-Cycloalkyl oder durch Halogen, -$OR_8$, -$OR_9$, -$SR_8$, -$SR_9$ oder $C_1$-$C_4$-Alkyl substituiertes $C_3$-$C_6$-Cycloalkyl steht, sowie die Salze dieser Verbindungen.

In den Definitionen der Substituenten ist unter $C_1$-$C_4$-Alkyl geradkettiges oder verzweigtes Alkyl zu verstehen, wie beispielsweise Methyl, Ethyl, n-Propyl, iso-Propyl, n-Butyl, sek.-Butyl, iso-Butyl und tert.-Butyl. Vorzugsweise besitzen die als oder in den Substituenten vorhandenen Alkylgruppen 1 bis 2 Kohlenstoffatome. Besonders bevorzugt ist Methyl.

Die in den Substituenten $R_6$ und $R_7$ vorkommenden $C_2$-$C_4$-Alkenylreste können geradkettig oder verzweigt sein. Bevorzugt sind Alkenylreste mit einer Kettenlänge von zwei bis drei Kohlenstoffatomen. Beispiele für $C_2$-$C_4$-Alkenylreste sind: Vinyl, Allyl, Methallyl, 1-Methylvinyl, But-2-en-1-yl. Bevorzugt ist Vinyl und Allyl.

Die in den Definitionen der Substituenten $R_6$ und $R_7$ vorkommenden $C_2$-$C_4$-Alkinylreste können geradkettig oder verzweigt sein. Bevorzugt sind Alkinylreste mit einer Kettenlänge von 2 bis 3 Kohlenstoffatomen. $C_2$-$C_4$-Alkinylreste stehen beispielsweise für Ethinyl, Propargyl, 1-Propinyl, 3-Butinyl oder 1-Methylpropargyl, wobei Ethinyl und Propargyl besonders bevorzugt sind.

Unter Halogen selbst sowie als Teil eines Substituenten wie in Halogenalkyl sind Fluor, Chlor und Brom, vorzugsweise jedoch Fluor und Chlor zu verstehen. Halogenalkyl steht in der Regel für Chlormethyl, Fluormethyl, Difluormethyl, Trifluormethyl, 2-Chlorethyl, 2,2,2-Trifluorethyl, 1,1,2,2-Tetrafluorethyl, Pentafluorethyl, 1,1,2-Trifluor-2-chlorethyl, 2,2,2-Trifluor-1,1-dichlorethyl, Pentachlorethyl, 3,3,3-Trifluorpropyl, 2,3-Dichlorpropyl, 1,1,2,3,3,3-Hexafluorpropyl, insbesondere aber Fluormethyl, Chlormethyl, Difluormethyl und Trifluormethyl.

Die in den Definitionen der Substituenten X,Y und Z vorkommenden $C_3$-$C_6$-Cycloalkylgruppen können substituiert oder unsubstituiert sein und umfassen beispielsweise Cyclopropyl, 2-Fluorcyclopropyl, 2,4-Difluorcyclopropyl, 2-Chlorcyclopropyl, 2,3-Dichlorcyclopropyl, 2-Methylcyclopropyl, 2-Methylthiocyclopropyl, 2,3-Dimethylcyclopropyl, 2-Methoxycyclopropyl, Cyclobutyl, Cyclopentyl, 3-Methylcyclopentyl, 3,4-Dimethoxycyclopentyl, Cyclohexyl, 3-Fluorcyclohexyl, 4-Methylcyclohexyl oder 4-Methylthiocyclohexyl.

Die Erfindung umfasst ebenfalls die Salze, die die Verbindungen der Formel I mit Aminen, Alkali- und Erdalkalimetallbasen bilden können.

Unter Alkali- und Erdalkalimetallhydroxiden als Salzbildner sind die Hydroxide von Lithium, Natrium, Kalium, Magnesium oder Calcium hervorzuheben, insbesondere aber die von Natrium oder Kalium.

Beispiele für zur Salzbildung geeignete Amine sind primäre, sekundäre und tertiäre aliphatische und aromatische Amine wie Methylamin, Ethylamin, Propylamin, iso-Propylamin, die vier isomeren Butylamine, Dimethylamin, Diethylamin, Diethanolamin, Dipropylamin, Diisopropylamin, Di-n-butylamin, Pyrrolidin, Piperidin, Morpholin, Trimethylamin, Triethylamin, Tripropylamin, Chinuclidin, Pyridin, Chinolin und iso-Chinolin, insbesondere aber Ethyl-, Propyl-, Diethyl- oder Triethylamin, vor allem aber iso-Propylamin und Diethanolamin.

Unter den Verbindungen der Formel I sind diejenigen bevorzugt, in denen

$R_1$ für Halogen, Nitro, $C_1$-$C_4$-Alkyl, $C_1$-$C_4$-Halogenalkyl, -$OR_8$, -$OR_9$, Hydroxy, Cyano, oder -$COOR_7$;

$R_5$ für Wasserstoff, Halogen, Nitro, $C_1$-$C_4$-Alkyl, $C_1$-$C_4$-Halogenalkyl, -$OR_8$, -$OR_9$, Hydroxy, Cyano oder -$COOR_7$; und

X, Y und Z unabhängig voneinander für Wasserstoff, $C_1$-$C_4$-Alkyl, $C_1$-$C_4$-Halogenalkyl, durch -$OR_9$ substituiertes $C_1$-$C_4$-Alkyl, durch -$OR_8$ substituiertes $C_1$-$C_4$-Alkyl, Halogen, $C_3$-$C_6$-Cycloalkyl oder durch Halogen, -$OR_8$, -$OR_9$, -$SR_8$, -$SR_9$ oder $C_1$-$C_4$-Alkyl substituiertes $C_3$-$C_6$-Cycloalkyl stehen.

In einer besonders bevorzugten Gruppe von Verbindungen der Formel I steht

$R_1$ für Halogen, Nitro, $C_1$-$C_2$-Alkyl, $C_1$-$C_2$-Halogenalkyl, -$OR_8$, -$OR_9$, Hydroxy, Cyano oder -$COOR_7$,

$R_2$ und $R_4$ unabhängig voneinander für Wasserstoff, Halogen, $C_1$-$C_2$-Alkyl, -$COOR_7$, oder -$OR_9$;

$R_5$ für Wasserstoff, Halogen, Nitro, $C_1$-$C_2$-Alkyl, $C_1$-$C_2$-Halogenalkyl, -$OR_8$, -$OR_9$, Hydroxy, Cyano, -$COOR_7$;

$R_7$ für $C_1$-$C_2$-Alkyl, $C_2$-$C_3$-Alkenyl, $C_2$-$C_3$-Alkinyl, Phenyl oder Benzyl;

$R_8$ für $C_1$-$C_2$-Halogenalkyl;

$R_9$ für $C_1$-$C_2$-Alkyl; und

X, Y und Z unabhängig voneinander für Wasserstoff, $C_1$-$C_2$-Alkyl, $C_1$-$C_2$-Halogenalkyl, Halogen, durch -$OR_9$ substituiertes $C_1$-$C_4$-Alkyl, durch -$OR_8$ substituiertes $C_1$-$C_4$-Alkyl, $C_3$-$C_6$-Cycloalkyl oder durch Halogen, -$OR_8$, -$OR_9$ oder $C_1$-$C_2$-Alkyl substituiertes $C_3$-$C_6$-Cycloalkyl.

Aus dieser bevorzugten Gruppe sind besonders diejenigen Verbindungen hervorzuheben, in denen

$R_1$ für Fluor, Chlor, Methyl, Trifluormethyl oder Methoxy;

$R_2$ und $R_4$ unabhängig voneinander für Wasserstoff, Fluor, Chlor, Methyl oder Methoxy;

$R_3$ für Wasserstoff;

$R_5$ für Wasserstoff, Fluor, Chlor, Methyl, Trifluormethyl oder Methoxy; und

X, Y und Z unabhängig voneinander für Wasserstoff, Methyl, Cyclopropyl, 2-Fluorcyclopropyl, 2,3-Difluorcyclopropyl, 2-Chlor-cyclopropyl, 2,3-Dichlorcyclopropyl, 1-Methylcyclopropyl, 2-Methylcyclopropyl, 2,3-Dimethylcyclopropyl, Cyclopentyl, Fluor, Chlor, Trifluormethyl oder Methoxy stehen.

Aus den Verbindungen der Formel I ragen durch ihre gute Wirksamkeit besonders diejenigen Verbindungen heraus, in denen X für $C_3$-$C_6$-Cycloalkyl oder durch Halogen, $-OR_8$, $-OR_9$, $-SR_8$, $-SR_9$ oder $C_1$-$C_4$-Alkyl substituiertes $C_3$-$C_6$-Cycloalkyl, vorzugsweise für Cyclopropyl, 1-Methylcyclopropyl, 2-Fluorcyclopropyl, 2,3-Difluorcyclopropyl, 2-Chlorcyclopropyl, 2,3-Dichlorcyclopropyl, 2-Methylcyclopropyl oder 2,3-Dimethylcyclopropyl, insbesondere aber für Cyclopropyl steht.

Eine bevorzugte Untergruppe der erfindungsgemässen Verbindungen der Formel I bilden die Verbindungen der Formel Ia

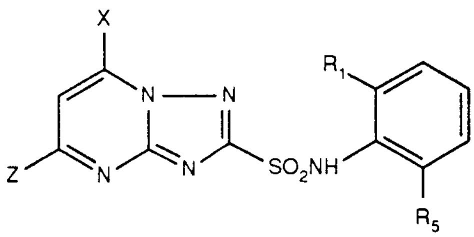

(Ia)

in welcher

$R_1$ für Halogen, Phenyl, O-Phenyl, $C_1$-$C_4$-Alkyl, $C_1$-$C_4$-Halogenalkyl, $-OR_8$, $-OR_9$, Nitro, Hydroxy, Cyano, $-SR_6$, $-SOR_6$, $-SO_2R_6$, $-SR_8$, $-SOR_8$, $-SO_2R_8$, $-SO_3R_8$, $-SR_9$, $SOR_9$, $-SO_2R_9$, $-SO_2NR_{10}(R_{10})$, $-SO_3R_9$, $-COOR_7$, $-CONHR_9$, $-CONR_9(R_9)$, Formyl,

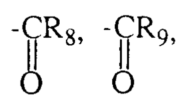

$-NH_2$, $NHR_9$ oder $-NR_9(R_9)$;

$R_5$ für Wasserstoff, Halogen, Phenyl, O-Phenyl, $C_1$-$C_4$-Alkyl, $C_1$-$C_4$-Halogenalkyl, $-OR_8$, $-OR_9$, Nitro, Hydroxy, Cyano, $SR_6$, $-SOR_6$, $-SO_2R_6$, $-SR_8$, $-SOR_8$, $-SO_3R_8$, $-SR_9$, $SOR_9$, $-SO_2R_9$, $-SO_2NR_{10}(R_{10})$, $-SO_3R_9$, $-COOR_7$, $-CONHR_9$, $-CONR_9(R_9)$, Formyl,

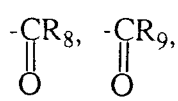

$-NH_2$, $NHR_9$ oder $-NR_9(R_9)$;

$R_6$ für Wasserstoff, Phenyl, Benzyl, $C_2$-$C_4$-Alkenyl, $C_2$-$C_4$-Alkinyl oder durch $-OR_9$ substituiertes $C_1$-$C_4$-Alkyl;

$R_7$ für $C_1$-$C_4$-Alkyl, $C_2$-$C_4$-Alkenyl, $C_2$-$C_4$-Alkinyl, Phenyl oder Benzyl;

$R_8$ für $C_1$-$C_4$-Halogenalkyl;

$R_9$ für $C_1$-$C_4$-Alkyl;

$R_{10}$ für Wasserstoff oder $C_1$-$C_4$-Alkyl;

X und Z unabhängig voneinander für Wasserstoff, $C_1$-$C_4$-Alkyl, $C_1$-$C_4$-Halogenalkyl, Phenylthio, Benzylthio, Hydroxy, Halogen, $-OR_8$, $-OR_9$, durch $-OR_9$ substituiertes $C_1$-$C_4$-Alkyl, durch $-OR_8$ substituiertes $C_1$-$C_4$-Alkyl, $-NH_2$, $-NHR_9$, $-NR_9(R_9)$, $C_3$-$C_6$-Cycloalkyl oder durch Halogen, $-OR_8$, $-OR_9$, $-SR_8$, $-SR_9$, oder $C_1$-$C_4$-Alkyl substituiertes $C_3$-$C_6$-Cycloalkyl stehen,

mit der Massgabe, dass mindestens einer der Substituenten X oder Z für $C_3$-$C_6$-Cycloalkyl oder durch Halogen, $-OR_8$, $-OR_9$, $-SR_8$, $-SR_9$ oder $C_1$-$C_4$-Alkyl substituiertes $C_3$-$C_6$-Cycloalkyl steht.

Von den Verbindungen der Formel Ia sind diejenigen bevorzugt in denen

$R_1$ für Halogen, Nitro, $C_1$-$C_4$-Alkyl, $C_1$-$C_4$-Halogenalkyl, $-OR_8$, $-OR_9$, Hydroxy, Cyano, oder $-COOR_7$;

$R_5$ für Wasserstoff, Halogen, Nitro, $C_1$-$C_4$-Alkyl, $C_1$-$C_4$-Halogenalkyl, $-OR_8$, $-OR_9$, Hydroxy, Cyano oder $-COOR_7$; und

X und Z unabhängig voneinander für Wasserstoff, $C_1$-$C_4$-Alkyl, $C_1$-$C_4$-Halogenalkyl, durch $-OR_9$ substituier-

tes $C_1$-$C_4$-Alkyl, durch -$OR_8$ substituiertes $C_1$-$C_4$-Alkyl, Halogen, $C_3$-$C_6$-Cycloalkyl oder durch Halogen, -$OR_8$, -$OR_9$, -$SR_8$, -$SR_9$ oder $C_1$-$C_4$-Alkyl substituiertes $C_3$-$C_6$-Cycloalkyl stehen.

Durch ihre gute biologische Wirksamkeit fallen besonders diejenigen Verbindungen der Formel Ia ins Auge, in denen

$R_1$ für Halogen, Nitro, $C_1$-$C_4$-Alkyl, $C_1$-$C_4$-Halogenalkyl, -$OR_8$, -$OR_9$, Hydroxy, Cyano, oder -$COOR_7$;

$R_5$ für Wasserstoff, Halogen, Nitro, $C_1$-$C_4$-Alkyl, $C_1$-$C_4$-Halogenalkyl, -$OR_8$, -$OR_9$, Hydroxy, Cyano oder -$COOR_7$; und

X und Z unabhängig voneinander für Wasserstoff, $C_1$-$C_4$-Alkyl, $C_1$-$C_4$-Halogenalkyl, Halogen, $C_3$-$C_6$-Cycloalkyl oder durch Halogen, -$OR_8$, -$OR_9$, -$SR_8$, -$SR_9$ oder $C_1$-$C_4$-Alkyl substituiertes $C_3$-$C_6$-Cycloalkyl stehen.

Aus dieser Gruppe sind diejenigen Verbindungen der Formel Ia bevorzugt, in denen

$R_1$ für Halogen, Nitro, $C_1$-$C_2$-Alkyl, $C_1$-$C_2$-Halogenalkyl, -$OR_8$, -$OR_9$, Hydroxy, Cyano oder -$COOR_7$,

$R_5$ für Wasserstoff, Halogen, Nitro, $C_1$-$C_2$-Alkyl, $C_1$-$C_2$-Halogenalkyl, -$OR_8$, -$OR_9$, Hydroxy, Cyano, -$COOR_7$;

$R_7$ für $C_1$-$C_2$-Alkyl, $C_2$-$C_3$-Alkenyl, $C_2$-$C_3$-Alkinyl, Phenyl oder Benzyl;

$R_8$ für $C_1$-$C_2$-Halogenalkyl;

$R_9$ für $C_1$-$C_2$-Alkyl; und

X und Z unabhängig voneinander für Wasserstoff, $C_1$-$C_2$-Alkyl, $C_1$-$C_2$-Halogenalkyl, Halogen, $C_3$-$C_6$-Cycloalkyl oder durch Halogen, -$OR_8$, -$OR_9$ oder $C_1$-$C_2$-Alkyl substituiertes $C_3$-$C_6$-Cycloalkyl stehen.

In einer weiteren hervorzuhebenden Untergruppe von Verbindungen der Formel Ia steht

$R_1$ für Fluor, Chlor, Methyl, Trifluormethyl oder Methoxy;

$R_5$ für Wasserstoff, Fluor, Chlor, Methyl, Trifluormethyl oder Methoxy; und

X und Z unabhängig voneinander für Wasserstoff, Methyl, Cyclopropyl, 1-Methylcyclopropyl, 2-Fluorcyclopropyl, 2,3-Difluorcyclopropyl, 2-Chlorcyclopropyl, 2,3-Dichlorcyclopropyl, 2-Methylcyclopropyl, 2,3-Dimethylcyclopropyl, Cyclopentyl, Fluor, Chlor, oder Methoxy.

Ganz besonders herausragende Gruppen von Verbindungen der Formel Ia sind dadurch charakterisiert, dass

$R_1$ für Fluor, Chlor, Methyl oder Trifluormethyl;

$R_5$ für Wasserstoff, Chlor, Methyl oder Trifluormethyl; und

X und Z unabhängig voneinander für Wasserstoff, Cyclopropyl, 1-Methylcyclopropyl, 2-Fluorcyclopropyl, 2,3-Difluorcyclopropyl, 2-Chlorcyclopropyl, 2,3-Dichlorcyclopropyl, 2-Methylcyclopropyl, 2,3-Dimethylcyclopropyl, Methyl, Methoxy, Fluor oder Chlor stehen.

Von dieser bevorzugten Untergruppe ragen insbesondere diejenigen Verbindungen durch ihre gute biologische Wirkung heraus, in denen X für Cyclopropyl steht.

Als bevorzugte Einzelverbindungen aus dem Umfang der Formel I sind zu nennen:

5-Methyl-7-cyclopropyl-N-(2,6-dichlorphenyl)-1,2,4-triazolo[1,5-a]pyrimidin-2-sulfonamid,
5-Methyl-7-cyclopropyl-N-(2,6-difluorphenyl)-1,2,4-triazolo[1,5-a]pyrimidin-2-sulfonamid,
7-Methyl-5-cyclopropyl-N-(2,6-dichlorphenyl)-1,2,4-triazolo[1,5-a]pyrimidin-2-sulfonamid,
5-Cyclopropyl-7-trifluormethyl-N-(2,6-dichlorphenyl)-1,2,4-triazolo[1,5-a]pyrimidin-2-sulfonamid, sowie
5-Methyl-7-cyclobutyl-N-(2,6-dichlorphenyl)-1,2,4-triazolo[1,5-a]pyrimidin-2-sulfonamid.

Die Verbindungen der Formel I werden hergestellt, indem man ein primäres Amin der Formel II

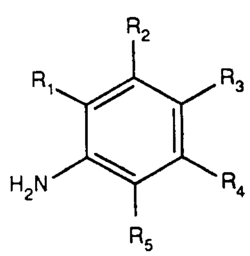

(II)

worin $R_1$, $R_2$, $R_3$, $R_4$ und $R_5$ die unter Formel I gegebene Bedeutung haben, in Gegenwart einer Base mit einem Triazolopyrimidinylsulfonylchlorid der Formel III

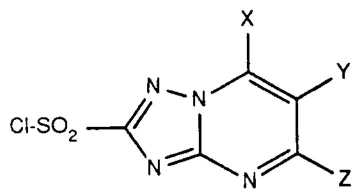

(III)

worin X, Y und Z die unter Formel I gegebenen Bedeutungen haben, umsetzt.

Das erfindungsgemässe Verfahren wird mit Vorteil in einem inerten Lösungsmittel bei einer Temperatur zwischen -20 ° C und der Siedetemperatur des Reaktionsgemisches durchgeführt. Ueblichersweise liegen die Temperaturen zwischen +15 ° C und +120 ° C, vorzugsweise zwischen +20 ° C und +80 ° C. Geeignete Lösungsmittel sind chlorierte Kohlenwasserstoffe, wie Methylenchlorid, Chloroform, Tetrachlorkohlenstoff, Trichlorethan, Tetrachlorethan, Chlorbenzol oder Dichlorbenzol; aromatische Kohlenwasserstoffe wie Benzol, Toluol oder Xylol; Ether wie Diethylether, Diisopropylether, Tetrahydrofuran, Ethylenglykoldimethylether oder Dioxan; Nitrile wie Acetonitril oder Propionitril; Cyclohexan oder Pyridin. Die Umsetzungen verlaufen im allgemeinen leicht exotherm und können bei Raumtemperatur durchgeführt werden. Als Basen sind insbesondere tertiäre Amine wie Trimethylamin, Triethylamin, N-Methylmorpholin, Chinuclidin, Pyridin, 1,4-Diazabicyclo[2.2.2]-octan, 1,5-Diazabicyclo[4.3.0]non-5-en oder 1,8-Diazabicyclo[5.4.0]undec-7-en geeignet.

Die Endprodukte der Formel I können durch Einengen und/oder Verdampfen des Lösungsmittels isoliert und durch Umkristallisieren oder Zerreiben des festen Rückstandes in Lösungsmitteln, in denen sie sich nicht gut lösen, wie Ether, aromatischen Kohlenwasserstoffen oder chlorierten Kohlenwasserstoffen, gereinigt werden.

Die primären Amine der Formel II können durch Reduktionsverfahren aus den entsprechenden Nitroverbindungen der Formel IV

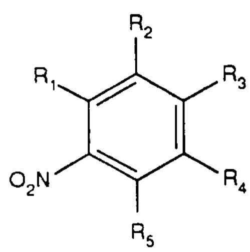

(IV)

worin $R_1$, $R_2$, $R_3$, $R_4$ und $R_5$ die unter Formel I gegebene Bedeutung haben, erhalten werden.

Zur Reduktion der Nitroverbindungen der Formel IV zu den Aminoverbindungen der Formel II sind alle gängigen in der Literatur beschriebenen Verfahren einsetzbar. Beispielsweise lässt sich die Reduktion mit Vorteil in wässrigem Medium in Gegenwart von Eisen, Zinn oder Zink und Salzsäure durchführen. Weitere geeignete Methoden sind Reduktionsverfahren unter Verwendung komplexer Hydride wie Lithiumaluminiumhydrid oder die katalytische Reduktion mit Wasserstoff unter Zuhilfenahme von Platin-, Palladium- oder Nickelkatalysatoren.

Die Nitroverbindungen der Formel IV können durch elektrophile Substitution in für aromatische Verbindungen üblichen Nitrierungsverfahren hergestellt werden. Die Zwischenprodukte der Formel II sind bekannt oder lassen sich analog bekannten Verbindungen herstellen.

Die Zwischenprodukte der Formel III sind neue Verbindungen und bilden ebenfalls einen Gegenstand der vorliegenden Erfindung. Die Zwischenprodukte der Formel III sind erhältlich durch Behandeln einer Verbindung der Formel V

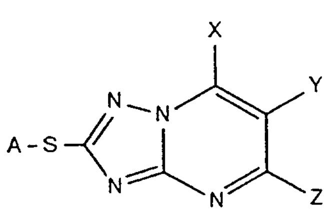

(V),

worin X, Y und Z die unter Formel I angegebene Bedeutung haben und A Wasserstoff, Isopropyl oder Benzyl bedeutet, mit der Massgabe, dass Y verschieden von Wasserstoff ist, wenn X und Z unabhängig voneinander Wasserstoff, $C_1$-$C_3$-Alkyl, Trifluormethyl oder Cyclopropyl sind, in wässrigem, saurem Medium mit Chlor. Als Säure verwendet man vorzugsweise Essigsäure oder anorganische Säuren, insbesondere Salzsäure. Die Behandlung mit Chlor erfolgt zweckmässigerweise bei Temperaturen im Bereich von -25 °C bis 20 °C, bevorzugt bei -15 °C bis 0 °C. Vorzugsweise erfolgt die Behandlung mit Chlor unter Zusatz von Dichlormethan zum Medium.

Die Zwischenprodukte der Formel V sind neue Verbindungen und bilden ebenfalls einen Gegenstand der vorliegenden Erfindung.Die Zwischenprodukte der Formel V können durch Umsetzung einer Verbindung der Formel VI

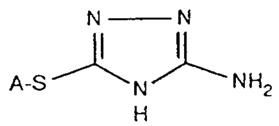

(VI),

worin A Wasserstoff oder Benzyl bedeutet, mit einer Verbindung der Formel VII

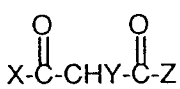

(VII),

worin X, Y und Z die unter Formel I angegebene Bedeutung haben, mit der Massgabe, dass Y verschieden von Wasserstoff ist, wenn X und Z unabhängig voneinander Wasserstoff, $C_1$-$C_3$-Alkyl, Trifluormethyl oder Cyclopropyl sind, hergestellt werden.

Die Verbindungen der Formel I können auch hergestellt werden, indem man eine Verbindung der Formel VIII

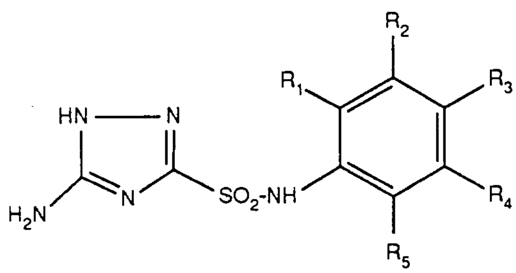

(VIII)

worin $R_1$, $R_2$, $R_3$, $R_4$ und $R_5$ die unter Formel I gegebene Bedeutung haben, mit einer Verbindung der Formel VII

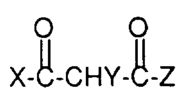

(VII)

worin X, Y und Z die unter Formel I angegebene Bedeutung haben, umsetzt. Derartige Verfahren sind beispielsweise in USP 4,734,123 beschrieben.

Die Zwischenprodukte der Formeln VI und VII sind bekannt oder lassen sich analog bekannten Methoden herstellen. Die Verbindung der Formel VI, worin A für Benzyl steht, lässt sich beispielsweise auf an sich bekannte Weise durch Umsetzung der Verbindung der Formel VI, in welcher A für Wasserstoff steht, mit Benzylchlorid herstellen.

Die Umsetzung einer Verbindung der Formel VI bzw. VIII mit einer Verbindung der Formel VII erfolgt vorteilhafterweise, indem man zunächst eine Verbindung der Formel VI bzw. VIII unter Erhitzen in wenig Eisessig löst und nach Zugabe der Verbindung der Formel VII das Reaktionsgemisch auf Rückflusstemperatur erhitzt.

Die Verbindungen der Formel I können ferner hergestellt werden, indem man ein Triazolopyrimidinylsulfonylchlorid der Formel III

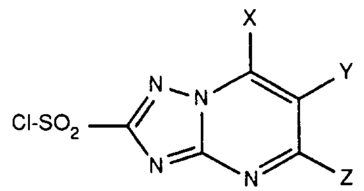

(III)

worin X, Y und Z die unter Formel I gegebenen Bedeutungen haben, mit einer Verbindung der Formel IX

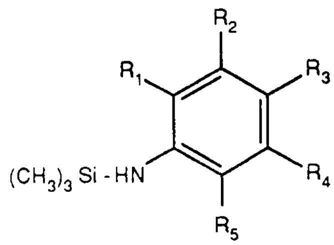

(IX)

worin $R_1$, $R_2$, $R_3$, $R_4$ und $R_5$ die unter Formel I gegebene Bedeutung haben, umsetzt. Derartige Verfahren sind beispielsweise in EP-A-0 343 752 beschrieben.

Die Wirkstoffe der Formel I werden in der Regel mit Aufwandmengen von 0,001 bis 5 kg/ha insbesondere 0,005 bis 3 kg/ha erfolgreich eingesetzt. Die für die erwünschte Wirkung erforderliche Dosierung kann durch Versuche ermittelt werden. Sie ist abhängig von der Art der Wirkung, dem Entwicklungsstadium der Kulturpflanze und des Unkrauts sowie von der Applikation (Ort, Zeit, Verfahren) und kann, bedingt durch diese Parameter, innerhalb weiter Bereiche variieren.

Bei geringeren Aufwandmengen zeichnen sich die Verbindungen der Formel I durch wuchshemmende und herbizide Eigenschaften aus, die sie ausgezeichnet zum Einsatz in Kulturen von Nutzpflanzen, insbesondere in Getreide, Baumwolle, Soja, Raps, Mais und Reis befähigen, wobei der Einsatz in Maiskultur ganz besonders bevorzugt ist.

Die Erfindung betrifft auch herbizide und pflanzenwachstumsregulierende Mittel, welche einen neuen Wirkstoff der Formel I enthalten, sowie Verfahren zur Hemmung des Pflanzenwuchses.

Pflanzenwachstumsregulatoren sind Substanzen, die in/an der Pflanze agronomisch erwünschte biochemische und/oder physiologische und/oder morphologische Veränderungen bewirken.

Die in den erfindungsgemässen Mitteln enthaltenen Wirkstoffe beeinflussen das Pflanzenwachstum je nach Applikationszeitpunkt, Dosierung, Applikationsart und Umweltbedingungen in verschiedener Weise. Pflanzenwuchsregulatoren der Formel I können zum Beispiel das vegetative Wachstum von Pflanzen hemmen. Diese Art der Wirkung ist von Interesse auf Rasenflächen, im Zierpflanzenbau, in Obstplantagen, an Strassenböschungen, auf Sport- und Industrieanlagen, aber auch bei der gezielten Hemmung von Nebentrieben wie bei Tabak. Im Ackerbau führt die Hemmung des vegetativen Wachstums bei Getreide über eine Halmverstärkung zu reduziertem Lager, ähnliche agronomische Wirkung erreicht man in Raps, Sonnenblumen, Mais und anderen Kulturpflanzen. Des weiteren kann durch Hemmung des vegetativen Wachstums die Anzahl Pflanzen pro Fläche erhöht werden. Ein weiteres Einsatzgebiet von Wuchshemmern ist die selektive Kontrolle von bodendeckenden Pflanzen in Plantagen oder weitreihigen Kulturen durch starke Wuchshemmung, ohne diese Bodendecker abzutöten, sodass die Konkurrenz gegenüber der Hauptkultur ausgeschaltet ist, die agronomisch positiven Effekte wie Erosionsverhinderung, Stickstoffbindung und Bodenlockerung aber erhalten bleiben.

Unter einem Verfahren zur Hemmung des Pflanzenwachstums ist eine Steuerung der natürlichen Pflanzenentwicklung zu verstehen, ohne den von genetischen Eigenschaften determinierten Lebenszyklus der Pflanze im Sinne einer Mutation zu verändern. Das Verfahren der Wuchsregulierung wird zu einem im Einzelfall zu bestimmenden Entwicklungszeitpunkt der Pflanze angewendet. Die Applikation der Wirkstoffe der Formel I kann vor oder nach dem Auflaufen der Pflanzen erfolgen, beispielsweise bereits auf die Samen oder die Sämlinge, auf Wurzeln, Knollen, Stengel, Blätter, Blüten oder andere Pflanzenteile. Dies kann z.B. durch Aufbringen des Wirkstoffes selbst oder in Form eines Mittels auf die Pflanzen und/oder durch

Behandlung des Nährmediums der Pflanze (Erdboden) geschehen.

Für die Verwendung der Verbindung der Formel I oder sie enthaltender Mittel zur Regulierung des Pflanzenwachstums kommen verschiedene Methoden und Techniken in Betracht, wie beispielsweise die folgenden:

i) Samenbeizung

a) Beizung der Samen mit einem als Spritzpulver formulierten Wirkstoff durch Schütteln in einem Gefäss bis zur gleichmässigen Verteilung auf der Samenoberfläche (Trockenbeizung). Man verwendet dabei bis zu 4 g Wirkstoff der Formel I (bei einer 50%-igen Formulierung: bis zu 8,0 g Spritzpulver) pro 1 kg Saatgut.

b) Beizung der Samen mit einem Emulsionskonzentrat des Wirkstoffs oder mit einer wässrigen Lösung des als Spritzpulver formulierten Wirkstoffs der Formel I nach Methode a) (Nassbeizung).

c) Beizung durch Tauchen des Saatguts in einer Brühe mit bis zu 1000 ppm Wirkstoff der Formel I während 1 bis 72 Stunden und gegebenenfalls nachfolgendes Trocknen der Samen (Tauchbeizung, Seed soaking).

Die Beizung des Saatguts oder die Behandlung des angekeimten Sämlings sind naturgemäss die bevorzugten Methoden der Applikation, weil die Wirkstoffbehandlung vollständig auf die Zielkultur gerichtet ist. Man verwendet in der Regel 4,0 g bis 0,001 g Aktivsubstanz pro 1 kg Saatgut, wobei man je nach Methodik, die auch den Zusatz anderer Wirkstoffe oder Mikronährstoffe ermöglicht, von den angegebenen Grenzkonzentrationen nach oben oder unten abweichen kann (Wiederholungsbeize).

ii) Kontrollierte Wirkstoffabgabe

Der Wirkstoff wird in Lösung auf mineralische Granulatträger oder polymerisierte Granulate (Harnstoff/Formaldehyd) aufgezogen und trocknen gelassen. Gegebenenfalls kann ein Ueberzug aufgebracht werden (Umhüllungsgranulate), der es erlaubt, den Wirkstoff über einen bestimmten Zeitraum dosiert abzugeben.

Die Verbindungen der Formel I werden in unveränderter Form oder vorzugsweise als Mittel zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen, in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

Die Formulierungen, d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Ether und Ester, wie Ethanol, Ethylenglykol, Ethylenglykolmonomethyl- oder -ethylether, Ketone wie Cyclohexanon, stark polare Lösungsmittel wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes der Formel I nichtionogene, kation- und[ch]oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen seien die Alkali-, Erdalkali- oder gegebenenfalls substituierte Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Talgöl gewonnen werden können, genannt. Ferner sind auch die Fettsäure-methyl-taurinsalze zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfate oder -sulfonate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2-Sulfonsäuregruppen und einen Fettsäurerest mit 8 bis 22 C-Atomen. Alkylarylsulfonate sind z.B. die Na-, Ca- oder Triethanolaminsalze der Dodecylbenzolsulfonsäure, der Dibutylnaphthalinsulfonsäure oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Aethylenoxid-Adduktes oder Phospholipide in Frage.

Als nicht ionische Tenside kommen in erster Linie Polyglykoletherderivate von aliphatischen oder cycloaliphatischen Alkoholen, gesättigten oder ungesättigten Fettsäuren und Alkylphenolen in Frage, die 3 bis 10 Glykolethergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Aethylenglykolethergruppen und 10 bis 100 Propylenglykolethergruppen enthaltenden Polyethylenoxidaddukte an Polypropylenglykol, Aethylendiaminopolypropylenglykol und Alkylpolypropylenglykol mit 1 bis 10 Kohlenstoffatomen in der Alkylkette, die genannten Verbindungen enthalten üblicherweise pro Propylenglykol-Einheit 1 bis 5 Ethylenglykoleinheiten.

Als Beispiele nichtionischer Tenside seien Nonylphenolpolyethoxyethanole, Ricinusölpolyethylenglykolether, Polypropylen-polyethylenoxidaddukte` Tributylphenoxypolyethoxyethanol, Polyethylenglykol und Octylphenoxypolyethoxyethanol erwähnt.

Ferner kommen auch Fettsäureester von Polyoxyethylensorbitan wie das Polyoxyethylensorbitan-trioleat in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Aethylsulfate vor, z.B. das Stearyltrimethylammoniumchlorid oder das Benzyldi(2-chlorethyl)-ethylammoniumbromid.

Die in der Formulierungstechnik gebräuchlichen Tenside sind u.a. in folgenden Publikationen beschrieben:

"Mc Cutcheon's Detergents and Emulsifiers Annual" MC Publishing Corp., Ridgewood. New Jersey, 1981;

H. Stache, "Tensid-Taschenbuch", 2. Aufl., C. Hanser Verlag, München, Wien, 1981;

M. and J. Ash. "Encyclopedia of Surfactants", Vol. I-III, Chemical Publishing Co., New York, 1980-1981.

Die agrochemischen Zubereitungen enthalten in der Regel 0,1 bis 95 %, insbesondere 0,1 bis 80 % Wirkstoff der Formel I, 1 bis 99,9 % eines festen oder flüssigen Zusatzstoffes und 0 bis 25 %, insbesondere 0,1 bis 25 % eines Tensides.

Insbesondere setzen sich bevorzugte Formulierungen folgendermassen zusammen: (% = Gewichtsprozent).

| Emulgierbare Konzentrate: | |
|---|---|
| Aktiver Wirkstoff: | 1 bis 20 %, bevorzugt 5 bis 10 % |
| oberflächenaktive Mittel: | 5 bis 30 %, vorzugsweise 10 bis 20 % |
| flüssiges Trägermittel: | 50 bis 94 %, vorzugsweise 70 bis 85 % |

| Stäubemittel: | |
|---|---|
| Aktiver Wirkstoff:<br>festes Trägermittel: | 0,1 bis 10 %, vorzugsweise 0,1 bis 1 %<br>99,9 bis 90 %, vorzugsweise 99,9 bis 99 % |

| Suspensions-Konzentrate: | |
|---|---|
| Aktiver Wirkstoff:<br>Wasser:<br>oberflächenaktives Mittel: | 5 bis 75 %, vorzugsweise 10 bis 50 %<br>94 bis 24 %, vorzugsweise 88 bis 30 %<br>1 bis 40 %, vorzugsweise 2 bis 30 % |

| Benetzbares Pulver: | |
|---|---|
| Aktiver Wirkstoff:<br>oberflächenaktives Mittel:<br>festes Trägermittel: | 0,5 bis 90 %, vorzugsweise 1 bis 80 %<br>0,5 bis 20 %, vorzugsweise 1 bis 15 %<br>5 bis 99 %, vorzugsweise 15 bis 90 % |

| Granulate: | |
|---|---|
| Aktiver Wirkstoff:<br>festes Trägermittel: | 0,5 bis 30 %, vorzugsweise 3 bis 15 %<br>99,5 bis 70 %, vorzugsweise 97 bis 85 %. |

Während als Handelsware eher konzentrierte Mittel bevorzugt werden, verwendet der Endverbraucher in der Regel verdünnte Mittel. Die Anwendungsformen können bis hinab zu 0,001 % an Wirkstoff verdünnt werden. Die Aufwandmengen betragen in der Regel 0,001 bis 5 kg AS/ha, vorzugsweise 0,005 bis 3 kg AS/ha.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Formulierungsbeispiele:

Beispiel F 1: Formulierungsbeispiele für Wirkstoffe der Formel I (% = Gewichtsprozent)

| a) Spritzpulver | | | |
|---|---|---|---|
| | a) | b) | c) |
| Wirkstoff Nr.1.1 | 20 % | 50 % | 0,5 % |
| Na-Ligninsulfonat | 5 % | 5 % | 4 % |
| Na-Laurylsulfat | 3 % | - | - |
| Na-Diisobutylnaphthalinsulfonat | - | 6 % | 6 % |
| Octylphenolpolyethylenglykoläther (7-8 Mol AeO) | - | 2 % | 2 % |
| Hochdisperse Kieselsäure | 5 % | 27 % | 27 % |
| Kaolin | 67 % | 10 % | - |
| Natriumchlorid | - | - | 59,5 % |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| b) Emulsions-Konzentrat | | |
|---|---|---|
| | a) | b) |
| Wirkstoff Nr. 1.1 | 10 % | 1 % |
| Octylphenolpolyethylenglykolether (4-5 Mol AeO) | 3 % | 3 % |
| Ca-Dodecylbenzolsulfonat | 3 % | 3 % |
| Ricinusolpolyglykolether (36 Mol AeO) | 4 % | 4 % |
| Cyclohexanon | 30 % | 10 % |
| Xylolgemisch | 50 % | 79 % |

Aus diesem Konzentrat können durch Verdünnen mit Wasser Emulsionen jeder gewünschten Konzentration hergestellt werden.

| c) Stäubemittel | | |
|---|---|---|
| | a) | b) |
| Wirkstoff Nr. 1.2 | 0,1 % | 1 % |
| Talkum | 99,9 % | - |
| Kaolin | - | 99 % |

Man erhält anwendungsfertige Stäubemittel, indem der Wirkstoff mit dem Träger vermischt und auf einer geeigneten Mühle vermahlen wird.

| d) Extruder Granulat | | |
|---|---|---|
| | a) | b) |
| Wirkstoff Nr. 1.1 | 10 % | 1 % |
| Na-Ligninsulfonat | 2 % | 2 % |
| Carboxymethylcellulose | 1 % | 1 % |
| Kaolin | 87 % | 96 % |

Der Wirkstoff wird mit den Zusatzstoffen vermischt, vermahlen und mit Wasser angefeuchtet. Dieses Gemisch wird extrudiert und anschliessend im Luftstrom getrocknet.

| e) Umhüllungs-Granulat | |
|---|---|
| Wirkstoff Nr. 1.1 | 3 % |
| Polyethylenglykol (MG 200) | 3 % |
| Kaolin | 94 % |

Der fein gemahlene Wirkstoff wird in einem Mischer auf das mit Polyethylenglykol angefeuchtete Kaolin gleichmässig aufgetragen. Auf diese Weise erhält man staubfreie Umhüllungs-Granulate.

| f) Suspensions-Konzentrat | | |
|---|---|---|
| | a) | b) |
| Wirkstoff Nr. 1.2 | 40 % | 5 % |
| Ethylenglykol | 10 % | 10 % |
| Nonylphenolpolyethylenglykolether (15 Mol AeO) | 6 % | 1 % |
| Na-Ligninsulfonat | 10 % | 5 % |
| Carboxymethylcellulose | 1 % | 1 % |
| 37%-ige wässrige Formaldehyd-Lösung | 0,2 % | 0,2 % |
| Silikonöl in Form einer 75%-igen wässrigen Emulsion | 0,8 % | 0,8 % |
| Wasser | 32 % | 77 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

| g) Salzlösung | |
|---|---|
| Wirkstoff Nr. 1.2 | 5 % |
| Isopropylamin | 1 % |
| Octylphenolpolyethylenglykolether (78 Mol AeO) | 3 % |
| Wasser | 91 % |

Herstellungsbeispiele:

Beispiel H1: Herstellung von 2-Benzylthio-5-methyl-7-cyclopropyl-1,2,4-triazolo[1,5-a]pyrimidin (Verbindung Nr. 2.1) und 2-Benzylthio-5-cyclopropyl-7-methyl-1,2,4-triazolo[1,5-a]pyrimidin (Verbindung Nr. 2.2)

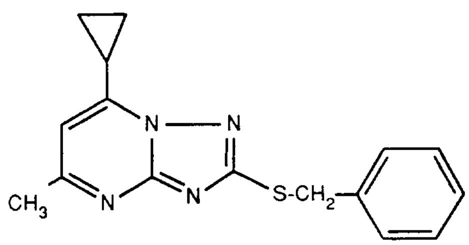

(2.1)

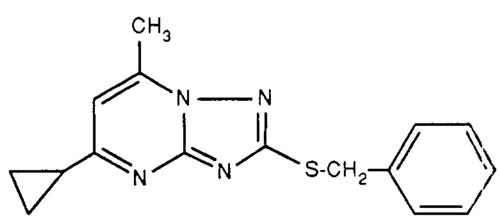

(2.2)

41,5 g 3-Amino-5-benzylthio-1,2,4-triazol werden in wenig heissem Eisessiggelöst und zusammen mit 30 g 1-Cyclopropyl-1,3-butandion am Rückfluss erhitzt. Nach ca. 1 Stunde wird die Reaktionslösung am Vakuum eingeengt und der Rückstand wird mit Wasser versetzt. Das ausgefallene dunkle Harz wird in Essigester aufgenommen, die Essigesterphase abgetrennt, über Natriumsulfat getrocknet und eingedampft. Der Rückstand wird an einer Kieselgelsäule (Laufmittel: Essigester/Hexan 1:1) gereinigt. Man erhält die Titelverbindungen A und B:

1. Fraktion 20 g Smp. +101 bis +103 °C (Verbindung Nr.2.2)
2. Fraktion 39 g Smp. +84 bis +86 °C (Verbindung Nr. 2.1)

Beispiel H2: Herstellung von 2-Benzylthio-7-cyclopropyl-1,2,4-triazolo[1,5-a]pyrimidin (Verbindung Nr. 2.20)

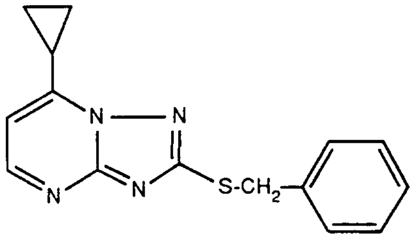

(2.20)

20 g 3-Amino-5-benzylthio-1,2,4-triazol werden mit 17 g 1-Cyclopropyl-1,3-butandion-Kaliumsalz (erhalten aus der Kondensationsreaktion von Acetylcyclopropan mit Ameisensäureethylester in Diethylether und Kalium-tert.-butylat) und 0,05 Mol Natriummethylat 3 Stunden lang in 300 ml absolutem Ethanol zum Sieden erhitzt. Anschliessend wird das Reaktionsgemisch am Vakuum eingeengt und der Rückstand mit Wasser versetzt. Nach Ansäuern mit konzentrierter Essigsäure wird das ausgefallene Produkt abgetrennt, getrocknet und aus Methylenchlorid/n-Hexan umkristallisiert. Man erhält 7 g 2-Benzylthio-7-cyclopropyl-1,2,4-triazolo[1,5-a]pyrimidin (Verbindung Nr. 2.20) mit einem Schmelzpunkt von +112 bis +114 °C.

Beispiel H3: Herstellung von 5-Cyclopropyl-7-methyl-1,2,4-triazolo[1,5-a]pyrimidin-2-yl-sulfochlorid (Verbindung Nr. 3.2)

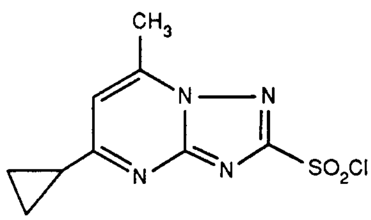

(3.2)

14 g 2-Benzylthio-5-cyclopropyl-7-methyl-1,2,4-triazolo[1,5-a]pyrimidin in 200 ml Dichlormethan und 200 ml Wasser werden mit 10 ml konzentrierter Salzsäure vermischt und kräftig gerührt. Bei 0 °C bis -3 °C werden im Zeitraum von ca. 20 Minuten 13,5 g Chlorgas eingeleitet. Nach etwa halbstündigem Nachrühren ohne Kühlung wird die organische Phase abgetrennt, mit Wasser nachgewaschen und über Natriumsulfat getrocknet. Nach dem Eindampfen am Vakuum verbleibt ein dunkles Oel, welches mehrmals mit Petrolether durchgerührt wird. Der Petrolether wird abdekantiert und der erhaltene zähe Rückstand getrocknet. Man erhält 11,5 g 5-Cyclopropyl-7-methyl-1,2,4-triazolo[1,5-a]pyrimidin-2-yl-sulfochiorid (Verbindung Nr. 3.2) als dunkles Oel (Rohprodukt), welches für die weiteren Umsetzungen geeignet ist. Das Protonenresonanzspektrum bestätigt die Konstitution der erhaltenen Verbindung.

Beispiel H4: Herstellung von 5-Methyl-7-cyclopropyl-1,2,4-triazolo[1,5-a]pyrimidin-2-yl-sulfochlorid (Verbindung Nr. 3.1)

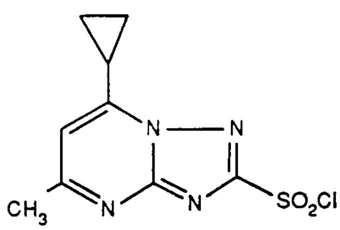

(3.1)

35 g 2-Benzylthio-5-methyl-7-cyclopropyl-1,2,4-triazolo[1,5-a]pyrimidin in 200 ml Dichlormethan werden mit 300 ml Wasser und 20 ml konzentrierter Salzsäure verrührt. In das Gemisch werden bei 0 °C bis -3 °C 33,5 g Chlorgas eingeleitet. Nach ca. 30 Minuten ist die Einleitung beendet und das Reaktionsgemisch wird noch ca. 20 Minuten ohne Kühlung nachgerührt. Nach Verdünnen mit Wasser wird die organische Phase abgetrennt, über Natriumsulfat getrocknet und am Vakuum eingedampft. Das erhaltene Oel wird mit mehreren Portionen Petroläther gewaschen. Nach dem Trocknen liegt 5-Methyl-7-cyclopropyl-1,2,4-triazolo-[1,5-a]pyrimidin-2-yl-sulfochlorid (Verbindung Nr. 3.1) als Rohprodukt vor, welches für die weiteren Umsetzungen geeignet ist. Ausbeute: 28 g dunkles Oel.

Beispiel H5: Herstellung von 5-Cyclopropyl-7-methyl-N-(2,6-dichlorphenyl)-1,2,4-triazolo[1,5-a]pyrimidin-2-sulfonamid (Verbindung Nr. 1.2)

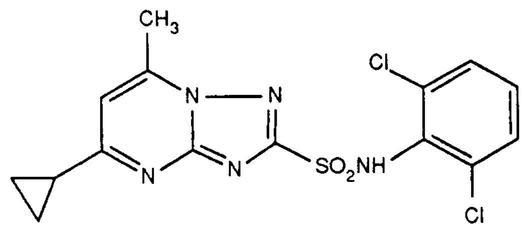

(1.2)

Eine Lösung aus 3,5 g 2,6-Dichloranilin in 15 ml wasserfreiem Pyridin wird mit 5,75 g 5-Cyclopropyl-7-methyl-1,2,4-triazolo[1,5-a]pyrmidin-2-ylsulfochlorid versetzt und anschliessend 20 Stunden lang bei Raumtemperatur gerührt. Nach Zugabe von 200 ml Wasser und 20 ml Essigsäureethylester stellt man den pH-Wert der Reaktionsmischung mit konzentrierter Salzsäure auf pH 3 ein. Das ausgefallene Produkt wird abfiltriert und der Filterrückstand mit Wasser und n-Hexan gewaschen. Man erhält nach dem Trocknen 6 g 5-Cyclopropyl-7-methyl-N-(2,6-dichlorphenyl)-1,2,4-triazolo[1,5-]pyrimidin-2-sulfonamid (Verbindung Nr. 1.2) mit einem Schmelzpunkt von +255 °C (Zers.).

Beispiel H6: Herstellung von 5-Methyl-7-cyclobutyl-N-(2,6-dichlorphenyl)-1,2,4-triazolo[1,5-a]pyrimidin-2-sulfonamid (Verbindung Nr. 1.25)

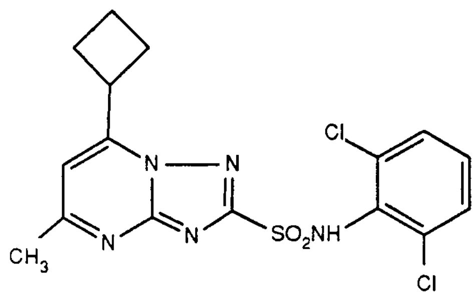

(1.25)

Unter Feuchtigkeitsausschluss werden 4,9 g 5-Methyl-7-cyclobutyl-1,2,4-triazolo[1,5-a]pyrimidin-2-yl-sulfochlorid in 50 ml Acetonitril gelöst und mit 4,2 g N-trimethylsilyl-2,6-dichloranilin und 0,2 ml Dimethylsulfoxid bis zum vollständigem Umsatz verrührt. Nach dem Filtrieren und Waschen mit Acetonitril wird das Filtrat eingedampft und anschliessend mit Wasser verrieben. Man erhält 1,8 g 5-Methyl-7-cyclobutyl-N-(2,6-dichlorphenyl)-1,2,4-triazolo[1,5-a]pyrimidin-2-sulfonamid (Verbindung Nr. 1.25) mit einem Schmelzpunkt von +260 C°.

Beispiel H7: Herstellung von 5-Methyl-7-cyclopentyl-N-(2,6-dichlorphenyl)-1,2,4-triazolo[1,5-a]pyrimidin-2-sulfonamid (Verbindung Nr. 1.85)

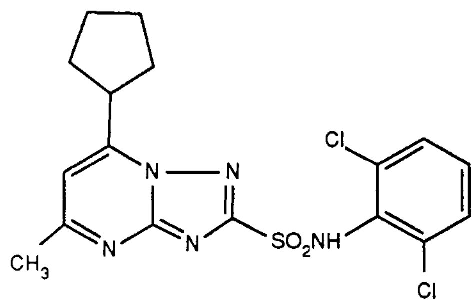

(1.85)

4g N-2,6-dichlorphenyl-5-amino-1,2,4-triazolo-3-yl-sulfonamid werden in 10 ml Dimethylsulfoxid und 15 ml Eisessig mit 4 g 1-Cyclopentyl-butan-1-3-dion 10 Stunden lang bei Raumtemperatur und 1 Stunde bei +100 °C verrührt. Nach Ausfällen des Produkts durch Zugabe von Wasser und Umkristallisieren aus Eisessig erhält man 4 g 5-Methyl-7-cyclopentyl-N-(2,6-dichlorphenyl)-1,2,4-triazolo[1,5-a]pyrimidin-2-sulfonamid (Verbindung Nr. 1.85) mit einem Schmelzpunkt von +245 bis +246 °C.

In analoger Weise werden die in den angeschlossenen Tabellen aufgelisteten Verbindungen der Formel I sowie die Zwischenprodukte der Formeln III und V hergestellt.

Tabelle 1:

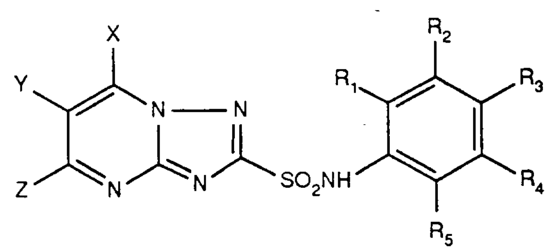

(I)

| Verb. Nr. | X | Y | Z | $R_1$ | $R_2$ | $R_3$ | $R_4$ | $R_5$ | Smp. [°C] |
|---|---|---|---|---|---|---|---|---|---|
| 1.1 | 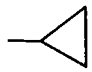 | H | $CH_3$ | Cl | H | H | H | Cl | +229 bis +231 (Zers.) |
| 1.2 | $CH_3$ | H | 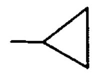 | Cl | H | H | H | Cl | +255 (Zers.) |
| 1.3 | 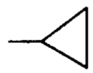 | H | 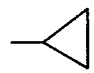 | Cl | H | H | H | Cl | +231 bis +234 (Zers.) |
| 1.4 | $CF_3$ | H | 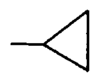 | Cl | H | H | H | Cl | +260 (Zers.) |
| 1.5 | 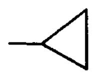 | H | H | Cl | H | H | H | Cl | +260 (Zers.) |
| 1.6 | 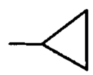 | H | $CF_3$ | Cl | H | H | H | Cl | |
| 1.7 | 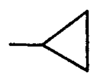 | H | $CH_2CH_3$ | Cl | H | H | H | Cl | |
| 1.8 | $CF_3$ | H | 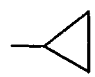 | Cl | H | H | H | Cl | |
| 1.9 | $CH_2CH_3$ | H | 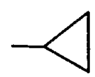 | Cl | H | H | H | Cl | +241 bis +244 |
| 1.10 | $C_3H_7$(n) | H | 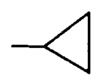 | Cl | H | H | H | Cl | |
| 1.11 | $C_3H_7$(i) | H | 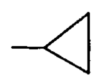 | Cl | H | H | H | Cl | +236 bis +238 |

Tabelle 1: (Fortsetzung)

| Verb. Nr. | X | Y | Z | $R_1$ | $R_2$ | $R_3$ | $R_4$ | $R_5$ | Smp. [°C] |
|---|---|---|---|---|---|---|---|---|---|
| 1.12 | $C_4H_9$(tert.) | H | 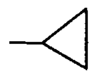 | Cl | H | H | H | Cl | +263 (Zers.) |
| 1.13 | $CH_2$-$OCH_3$ | H | 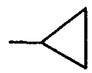 | Cl | H | H | H | Cl | |
| 1.14 | Cl | H | 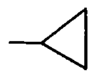 | Cl | H | H | H | Cl | |
| 1.15 | $OCH_3$ | H | 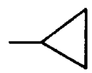 | Cl | H | H | H | Cl | |
| 1.16 | 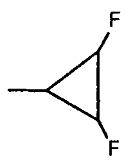 | H | H | Cl | H | H | H | Cl | |
| 1.17 | $CF_3$ | H | 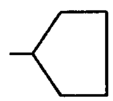 | Cl | H | H | H | Cl | |
| 1.18 | -$OC_2H_5$ | H | 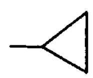 | Cl | H | H | H | Cl | |
| 1.19 | OH | H | 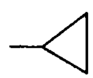 | Cl | H | H | H | Cl | |
| 1.20 | H | H | 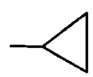 | Cl | H | H | H | Cl | |
| 1.21 | -$COOCH_3$ | H | 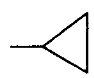 | Cl | H | H | H | Cl | |

Tabelle 1: (Fortsetzung)

| Verb. Nr. | X | Y | Z | $R_1$ | $R_2$ | $R_3$ | $R_4$ | $R_5$ | Smp. [°C] |
|---|---|---|---|---|---|---|---|---|---|
| 1.22 | 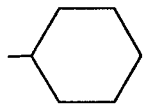 | H | $CH_2CH_3$ | Cl | H | H | H | Cl | |
| 1.23 | 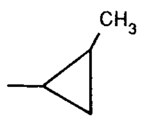 | H | $CH_3$ | Cl | H | H | H | Cl | |
| 1.24 | $OCH_2CHCl_2$ | H | 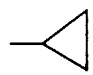 | Cl | H | H | H | Cl | |
| 1.25 | 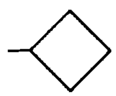 | H | $CH_3$ | Cl | H | H | H | Cl | |
| 1.26 | 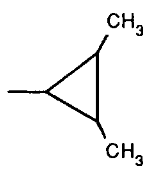 | H | $CH_3$ | Cl | H | H | H | Cl | |
| 1.27 | $CH_3$ | H | 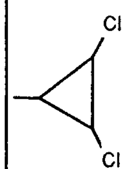 | Cl | H | H | H | Cl | |
| 1.28 | $NH_2$ | H | 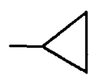 | Cl | H | H | H | Cl | |
| 1.29 | $NHCH_3$ | H | 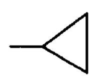 | Cl | H | H | H | Cl | |

Tabelle 1: (Fortsetzung)

| Verb. Nr. | X | Y | Z | $R_1$ | $R_2$ | $R_3$ | $R_4$ | $R_5$ | Smp. [°C] |
|---|---|---|---|---|---|---|---|---|---|
| 1.30 | $N(C_2H_5)_2$ | H | 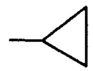 | Cl | H | H | H | Cl | |
| 1.31 | 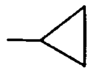 | H | $CH_3$ | Cl | H | H | H | $CH_3$ | |
| 1.32 | 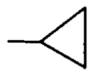 | H | $CF_3$ | Cl | H | H | H | F | |
| 1.33 | 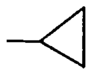 | H | $SCH_3$ | Cl | H | H | H | Cl | |
| 1.34 | 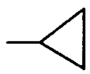 | H | Cl | Cl | H | H | H | $OCH_3$ | |
| 1.35 | 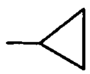 | H | $CH_3$ | Cl | H | H | H | $CF_3$ | |
| 1.36 | 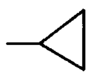 | $CH_3$ | $CH_3$ | Cl | H | H | H | $NO_2$ | |
| 1.37 | 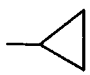 | Cl | $CH_3$ | Cl | H | H | H | OH | |
| 1.38 | $CH_3$ | H | 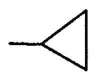 | $CH_3$ | $CH_3$ | H | $CH_3$ | H | |
| 1.39 | $CH_3$ | H | 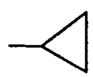 | F | H | Cl | $OCH_3$ | H | |
| 1.40 | $CH_3$ | H | 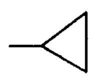 | Br | H | H | $COOCH_3$ | H | |

Tabelle 1: (Fortsetzung)

| Verb. Nr. | X | Y | Z | $R_1$ | $R_2$ | $R_3$ | $R_4$ | $R_5$ | Smp. [°C] |
|---|---|---|---|---|---|---|---|---|---|
| 1.41 | $CH_3$ | $CH_3$ | 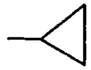 | H | Cl | H | H | Cl | |
| 1.42 | $CH_3$ | $CF_3$ | 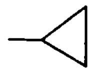 | H | H | F | 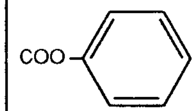 | Cl | |
| 1.43 | $CH_3$ | $OCH_3$ | 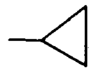 | H | H | Br | H | Cl | |
| 1.44 | $CH_3$ | 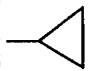 | 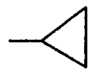 | H | H | H | H | CHO | |
| 1.45 | $CH_3$ | Br | 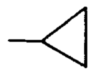 | H | H | H | H | CN | |
| 1.46 | $CH_3$ | H | 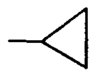 | H | H | H | H | $NH_2$ | |
| 1.47 | $CH_3$ | H | 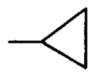 | Cl | H | H | H | $SCH_3$ | |
| 1.48 | $CH_3$ | H | 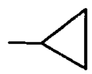 | Cl | H | H | H | $SOCH_3$ | |
| 1.49 | $CH_3$ | H | 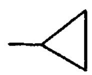 | Cl | H | H | H | $SO_2CH_3$ | |
| 1.50 | $CH_3$ | H | 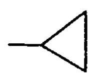 | Cl | H | H | H | $SO_3CH_3$ | |

Tabelle 1: (Fortsetzung)

| Verb. Nr. | X | Y | Z | $R_1$ | $R_2$ | $R_3$ | $R_4$ | $R_5$ | Smp. [°C] |
|---|---|---|---|---|---|---|---|---|---|
| 1.51 | $CH_3$ | H | 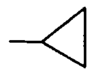 | Cl | H | H | H | $CONHCH_3$ | |
| 1.52 | $CH_3$ | H | 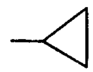 | Cl | H | H | H | $CON(CH_3)_2$ | |
| 1.53 | $CH_3$ | H | 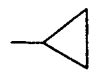 | Cl | H | H | H | 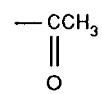 | |
| 1.54 | $CH_3$ | H | 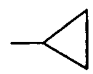 | Cl | H | H | H | $NHCH_3$ | |
| 1.55 | $CH_3$ | H | 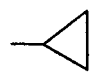 | Cl | H | H | H | $N(CH_2CH_3)_2$ | |
| 1.56 | H | H | 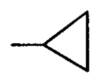 | F | H | H | H | H | |
| 1.57 | H | H | 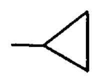 | 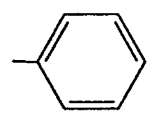 | H | H | H | H | |
| 1.58 | H | H | 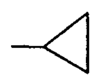 | 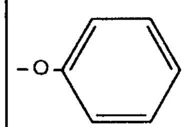 | H | H | H | H | |
| 1.59 | H | H | 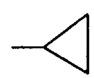 | $CH_3$ | H | H | H | H | |

Tabelle 1: (Fortsetzung)

| Verb. Nr. | X | Y | Z | $R_1$ | $R_2$ | $R_3$ | $R_4$ | $R_5$ | Smp. [°C] |
|---|---|---|---|---|---|---|---|---|---|
| 1.60 | H | 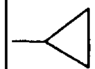 | $SCH_2CHCl_2$ | $C_2H_5$ | H | H | H | H | |
| 1.61 | H | 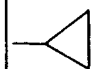 | $CH_3$ | $CF_3$ | H | H | H | H | |
| 1.62 | H | 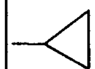 | $C_2H_5$ | $CH_2CF_3$ | H | H | H | H | |
| 1.63 | H | 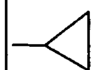 | $C_3H_7$(n) | $OCH_3$ | H | H | H | H | |
| 1.64 | H | 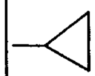 | $C_4H_9$(tert). | $OCH_2Cl$ | H | H | H | H | |
| 1.65 | 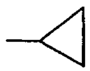 | H | H | $NO_2$ | H | H | H | H | |
| 1.66 | 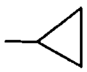 | H | H | OH | H | H | H | H | |
| 1.67 | 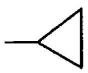 | H | H | CN | H | H | H | H | |
| 1.68 | 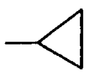 | $CH_3$ | H | $SCH_3$ | H | H | H | H | |
| 1.69 | 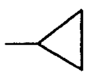 | $CF_3$ | H | $SOCH_3$ | H | H | H | H | |
| 1.70 | 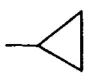 | $SCH_3$ | H | $SO_2C_2H_5$ | H | H | H | H | |
| 1.71 | $-CH_2-CH_2-CH_2-$ | | 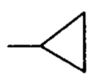 | Cl | H | H | H | Cl | |

Tabelle 1: (Fortsetzung)

| Verb. Nr. | X | Y | Z | $R_1$ | $R_2$ | $R_3$ | $R_4$ | $R_5$ | Smp. [°C] |
|---|---|---|---|---|---|---|---|---|---|
| 1.72 | 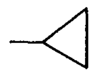 | H | $CH_3$ | Br | H | H | H | Br | +239 bis +241,5 |
| 1.73 | 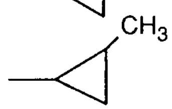 | H | $CH_3$ | F | H | H | H | F | +235 bis +237 |
| 1.74 | 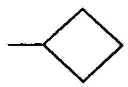 | H | $CH_3$ | Cl | H | H | $CH_3$ | Cl | +255 bis +257 |
| 1.75 | $-CH_2-OCH_3$ | H | 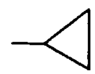 | Cl | H | H | $CH_3$ | Cl | +250 bis +254 |
| 1.76 | 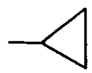 | H | $-CH_2-OCH_3$ | Cl | H | H | H | Cl | +194 bis +196 |
| 1.77 | $CH_3$ | H | 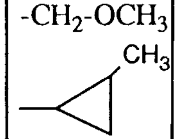 | Cl | H | H | H | Cl | +292,4 (Zers.) |
| 1.78 | $CH_3$ | H | 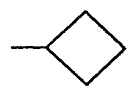 | Cl | H | H | H | Cl | +300 (Zers.) |
| 1.79 | 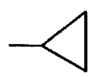 | $-CH_2-CH_2-CH_2-$ | | Cl | H | H | H | Cl | +245 bis +246 |
| 1.80 | 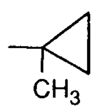 | H | $CH_3$ | Cl | H | H | H | Cl | +253 bis +256 |
| 1.81 | 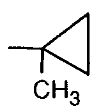 | H | $CH_3$ | F | H | H | H | F | +261 |

Tabelle 1: (Fortsetzung)

| Verb. Nr. | X | Y | Z | $R_1$ | $R_2$ | $R_3$ | $R_4$ | $R_5$ | Smp. [°C] |
|---|---|---|---|---|---|---|---|---|---|
| 1.82 | $CH_3$ | H | 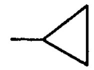 | F | H | H | H | F | +300 (Zers.) |
| 1.83 | 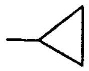 | H | $CH_3$ | F | H | H | H | F | +240 bis +241 (Zers.) |
| 1.84 | 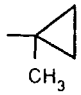 | H | H | Cl | H | H | H | Cl | +260 (Zers.) |
| 1.85 | 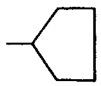 | H | $CH_3$ | Cl | H | H | H | Cl | +245 bis +246 |
| 1.86 | $OCH_3$ | H | 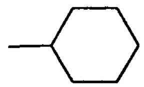 | Cl | H | H | H | Cl | +221 bis +226 |
| 1.87 | 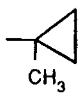 | H | $CH_3$ | Cl | $CH_3$ | H | H | Cl | >300 |
| 1.88 | 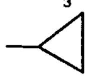 | H | $CH_3$ | Cl | $CH_3$ | H | H | Cl | +244 bis +246.5 |
| 1.89 | $CH_3$ | H | 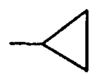 | Cl | $CH_3$ | H | H | Cl | +282 bis +286 |
| 1.90 | 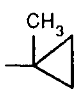 | H | $CH_3$ | Cl | $CH_3$ | H | H | Cl | >+300 |
| 1.91 | $CH_3$ | H | 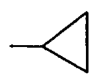 | Br | H | H | H | Br | >+250 |

Tabelle 1: (Fortsetzung)

| Verb. Nr. | X | Y | Z | $R_1$ | $R_2$ | $R_3$ | $R_4$ | $R_5$ | Smp. [°C] |
|---|---|---|---|---|---|---|---|---|---|
| 1.92 | 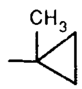 | H | H | Br | H | H | H | Br | >+300 |
| 1.93 | $OCH_3$ | H | 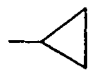 | Cl | H | H | H | Cl | +206 bis +209 |
| 1.94 | 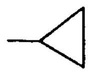 | H | $CH_3$ | $CF_3$ | H | H | H | H | +171 bis +174 |
| 1.95 | 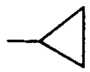 | H | $CH_3$ | Cl | H | H | H | H | +191.5 bis +173.5 |
| 1.96 | 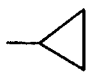 | H | $CH_3$ | Cl | H | H | $CF_3$ | H | +190.4 bis +143 |
| 1.97 | 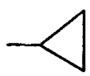 | H | $CH_3$ | H | H | Cl | $CF_3$ | H | +257 bis +259 |
| 1.98 | 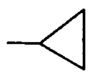 | H | $CH_3$ | F | H | Cl | 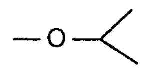 | H | +217.8 bis +219.5 |

Tabelle 2:

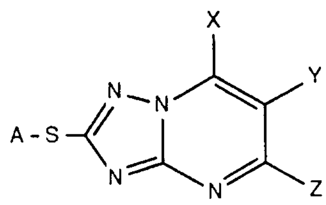

(V)

| Verb. Nr. | X | Y | Z | A | Smp. [°C] |
|---|---|---|---|---|---|
| 2.1 | 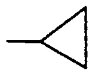 | H | $CH_3$ | Benzyl | +84 bis +86 |
| 2.2 | $CH_3$ | H | 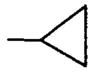 | Benzyl | +101 bis +103 |
| 2.3 | 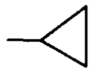 | H | 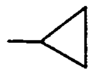 | Benzyl | +96 bis +98 |
| 2.4 | $CF_3$ | H | 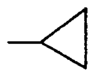 | Benzyl | +123 bis +124 |
| 2.5 | 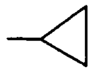 | H | H | Benzyl | +82 bis +86 |
| 2.6 | 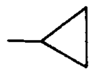 | H | $CF_3$ | Benzyl | |
| 2.7 | 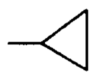 | H | $CH_2CH_3$ | Benzyl | +112 bis +114 |
| 2.8 | $CF_3$ | H | 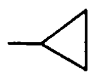 | H | |
| 2.9 | $CH_2CH_3$ | H | 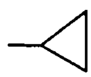 | Benzyl | +84 bis +85 |
| 2.10 | $C_3H_7$(n) | H | 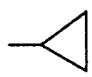 | Benzyl | |
| 2.11 | $C_3H_7$(i) | H | 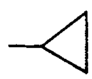 | Benzyl | |
| 2.12 | $C_4H_9$(tert.) | H | 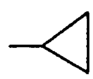 | Benzyl | +111 bis +113 |
| 2.13 | $CH_2$-$OCH_3$ | H | 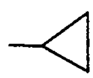 | Benzyl | |
| 2.14 | Cl | H | 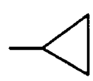 | Benzyl | |
| 2.15 | $OCH_3$ | H | 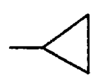 | Benzyl | |
| 2.16 | 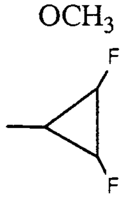 | H | H | Benzyl | |

Tabelle 2: (Fortsetzung)

| Verb. Nr. | X | Y | Z | A | Smp. [°C] |
|---|---|---|---|---|---|
| 2.17 | $CF_3$ | H | 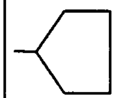 | Benzyl | |
| 2.18 | $-OC_2H_5$ | H | 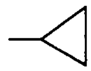 | Benzyl | |
| 2.19 | OH | H | 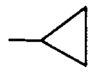 | Benzyl | |
| 2.20 | H | H | 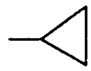 | Benzyl | +112 bis +114 |
| 2.21 | $-COOCH_3$ | H | 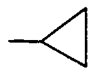 | Benzyl | |
| 2.22 | 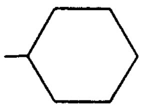 | H | $CH_2CH_3$ | Benzyl | |
| 2.23 | 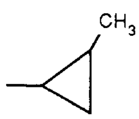 | H | $CH_3$ | Benzyl | |
| 2.24 | $OCH_2CHCl_2$ | H | 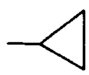 | Benzyl | |
| 2.25 | 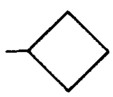 | H | $CH_3$ | Benzyl | |
| 2.26 | 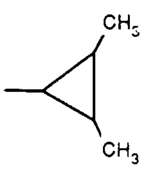 | H | $CH_3$ | Benzyl | |
| 2.27 | $CH_3$ | H | 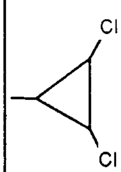 | Benzyl | |
| 2.28 | $NH_2$ | H | 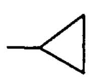 | Benzyl | |
| 2.29 | $NHCH_3$ | H | 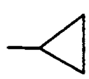 | Benzyl | |
| 2.30 | $N(C_2H_5)_2$ | H | 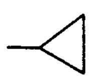 | Benzyl | |

Tabelle 2: (Fortsetzung)

| Verb. Nr. | X | Y | Z | A | Smp. [°C] |
|---|---|---|---|---|---|
| 2.31 | 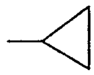 | H | $CH_3$ | H | |
| 2.32 | 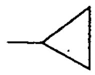 | H | $CF_3$ | H | |
| 2.33 | 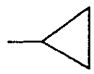 | H | $SCH_3$ | H | |
| 2.34 | 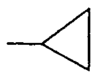 | H | Cl | H | |
| 2.35 | 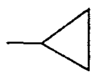 | $C_2H_5$ | $CH_3$ | H | |
| 2.36 | 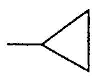 | $CH_3$ | $CH_3$ | H | |
| 2.37 | 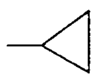 | Cl | $CH_3$ | H | |
| 2.38 | $CH_3$ | H | 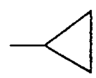 | H | |
| 2.39 | $CH_3$ | $C_3H_7$(i) | 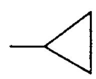 | H | |
| 2.40 | $CH_3$ | $OCH_2CH_3$ | 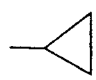 | H | |
| 2.41 | $CH_3$ | $CH_3$ | 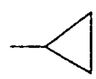 | H | |
| 2.42 | $CH_3$ | $CF_3$ | 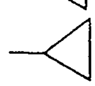 | H | |
| 2.43 | $CH_3$ | $OCH_3$ | 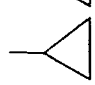 | H | |
| 2.44 | $CH_3$ | 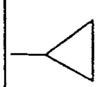 | 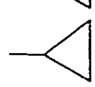 | H | |
| 2.45 | $CH_3$ | Br | 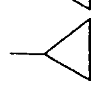 | H | |
| 2.46 | $-CH_2-CH_2-CH_2-$ | | 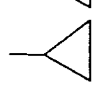 | Benzyl | +93 bis +94 |
| 2.47 | 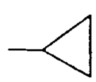 | $-CH_2-CH_2-CH_2-$ | | Benzyl | +113 bis +114 |

Tabelle 3:

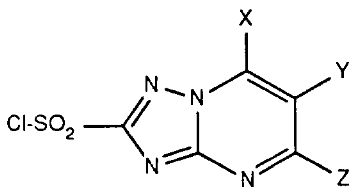

(III)

| Verb. Nr. | X | Y | Z | Smp. [°C] |
|---|---|---|---|---|
| 3.1 | 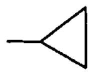 | H | $CH_3$ | Oel |
| 3.2 | $CH_3$ | H | 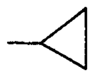 | Oel |
| 3.3 | 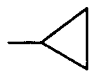 | H | 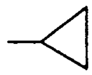 | +127 bis +130 |
| 3.4 | $CF_3$ | H | 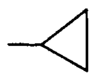 | +78 bis +81 |
| 3.5 | 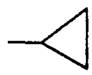 | H | H | +100 bis +103 |
| 3.6 | 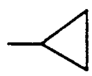 | H | $CF_3$ | |
| 3.7 | 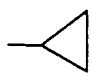 | H | $CH_2CH_3$ | +62 bis +63 |
| 3.8 | H | H | 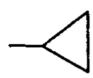 | +124 bis + 129 |
| 3.9 | $CH_2CH_3$ | H | 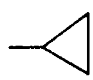 | +83 bis +86 |
| 3.10 | $C_3H_7$(n) | H | 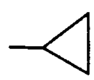 | |
| 3.11 | $C_3H_7$(i) | H | 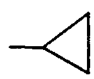 | +121 |
| 3.12 | $C_4H_9$(tert.) | H | 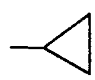 | +123 |
| 3.13 | $CH_2$-$OCH_3$ | H | 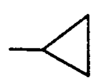 | |
| 3.14 | Cl | H | 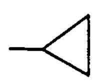 | +82 |
| 3.15 | $OCH_3$ | H | 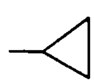 | +125 bis +130 |
| 3.16 | 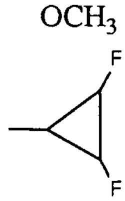 | H | H | |

Tabelle 3: (Fortsetzung)

| Verb. Nr. | X | Y | Z | Smp. [°C] |
|---|---|---|---|---|
| 3.17 | $CF_3$ | H | 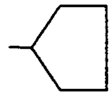 | |
| 3.18 | $-OC_2H_5$ | H | 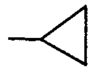 | |
| 3.19 | OH | H | 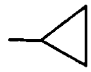 | |
| 3.20 | H | H | 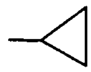 | |
| 3.21 | $-COOCH_3$ | H | 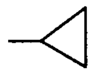 | |
| 3.22 | 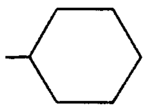 | H | $CH_2CH_3$ | |
| 3.23 | 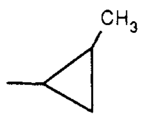 | H | $CH_3$ | |
| 3.24 | $OCH_2CHCl_2$ | H | 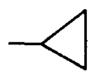 | |
| 3.25 | 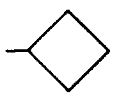 | H | $CH_3$ | |
| 3.26 | 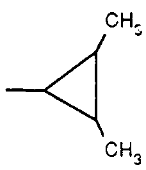 | H | $CH_3$ | |
| 3.27 | $CH_3$ | H | 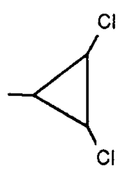 | |
| 3.28 | $NH_2$ | H | 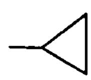 | |
| 3.29 | $NHCH_3$ | H | 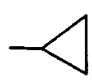 | |
| 3.30 | $N(C_2H_5)_2$ | H | 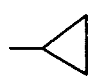 | |

Tabelle 3: (Fortsetzung)

| Verb. Nr. | X | Y | Z | Smp. [°C] |
|---|---|---|---|---|
| 3.31 | 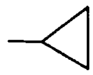 | H | $SCH_3$ | |
| 3.32 | 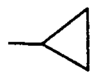 | $CH_3$ | $CH_3$ | |
| 3.33 | 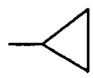 | Cl | $CH_3$ | |
| 3.34 | $CH_3$ | $CF_3$ | 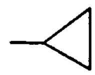 | |
| 3.35 | $CH_3$ | $OCH_3$ | 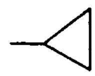 | |
| 3.36 | $CH_3$ | 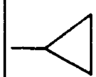 | 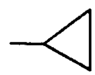 | |
| 3.36 | $CH_3$ | Br | 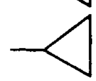 | |
| 3.37 | $-CH_2-CH_2-CH_2-$ | | 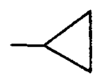 | +152 bis +153 |
| 3.38 | 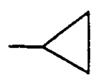 | $-CH_2-CH_2-CH_2-$ | | |
| 3.39 | 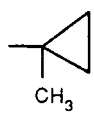 | H | H | +151 bis +156 |
| 3.40 | 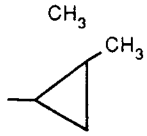 | H | $CH_3$ | Oel |
| 3.41 | $CH_3$ | H | 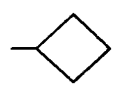 | +91 bis +95 |
| 3.42 | 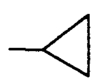 | H | $CH_2-OCH_3$ | +90 bis +95 |
| 3.43 | 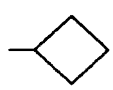 | H | $CH_3$ | +68 bis +75 |
| 3.44 | $CH_2-OCH_3$ | H | 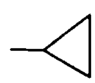 | +73 bis +77 |
| 3.45 | 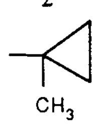 | H | $CH_3$ | |
| 3.46 | $OCH_3$ | H | 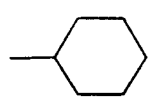 | |

Biologische Beispiele

Beispiel B1: Pre-emergente Herbizid-Wirkung

Im Gewächshaus wird unmittelbar nach der Einsaat der Versuchspflanzen in Saatschalen die Erdoberfläche mit einer wässrigen Spritzbrühe entsprechend einer Aufwandmenge von 4 kg Wirksubstanz/Hektar behandelt. Die Saatschalen werden im Gewächshaus bei 22-25°C und 50-70 % relativer Luftfeuchtigkeit gehalten.

Nach 3 Wochen wird die Herbizidwirkung mit einem neunstufigen (1 = vollständige Schädigung, 9 = keine Wirkung) Boniturschema im Vergleich in einer unbehandelten Kontrollgruppe bewertet.

Boniturnoten von 1 bis 4 (insbesondere 1 bis 3) weisen auf eine gute bis sehr gute Herbizidwirkung hin. Boniturnoten von 6 bis 9 (insbesondere von 7 bis 9) weisen auf eine gute Toleranz (insbesondere bei Kulturpflanzen) hin.

In diesem Versuch zeigen die Verbindungen der Tabelle 1 starke Herbizidwirkung.

Beispiel B2: Post-emergente Herbizid-Wirkung (Kontaktherbizid)

Eine Anzahl Unkräuter, sowohl monokotyle wie dikotyle, wurden nach dem Auflaufen (im 4- bis 6-Blattstadium) mit einer wässrigen Wirkstoffdispersion in einer Dosierung von 4 kg Wirksubstanz pro Hektar auf die Pflanzen gespritzt und diese bei 24°-26°C und 45-60 % relativer Luftfeuchtigkeit gehalten. 15 Tage nach der Behandlung wird der Versuch ausgewertet. Auch in diesem Versuch zeigen die Verbindungen der Tabelle 1 gute Herbizidwirkung.

Beispiel B3: Herbizidwirkung für Wasserreis (paddy)

Die Wasserunkräuter Echinochloa crus galli und Monocharia vag. werden in Plastikbechern (60 $cm^2$ Oberfläche, 500 ml Volumen) ausgesät. Nach der Saat wird bis zur Erdoberfläche mit Wasser aufgefüllt. 3 Tage nach der Saat wird der Wasserspiegel bis leicht über die Erdoberfläche erhöht (3-5 mm). Die Applikation erfolgt 3 Tage nach der Saat durch eine Spritzung der Prüfsubstanzen auf die Gefässe. Die verwendete Dosis entspricht einer Wirkstoffmenge von 4 kg AS pro Hektar. Die Pflanzenbecher werden dann im Gewächshaus unter optimalen Wachstumsbedingungen für die Reisunkräuter aufgestellt, d.h. bei 25°-30°C und hoher Luftfeuchtigkeit.

Die Auswertung der Versuche findet 3 Wochen nach Applikation statt. Die Verbindungen der Tabelle 1 schädigen dabei die Unkräuter, nicht aber den Reis.

Beispiel B4: Wuchshemmung bei tropischen Bodenbedecker-Leguminosen (cover crops)

Die Versuchspflanzen Centrosema pubescens und Psophocarpus palustris werden in 4 cm-Tontöpfen mit Landerde (45 %), Torf (45 %) und Zonolit (10 %) durch Stecklinge vermehrt. Die Anzucht erfolgt im Gewächshaus bei einer Tagestemperatur von 27°C und einer Nachttemperatur von 23°C. Die Beleuchtungsdauer ist mindestens 14 Stunden/Tag bei einer Intensität von mindestens 7000 Lux.

Ca. 50 Tage nach Ansatz der Stecklinge erfolgt das Vertopfen in 13 cm-Töpfe, 4-5 Pflanzen/Topf. Nach weiteren 60 Tagen werden die Pflanzen auf ca. 15 cm Höhe zurückgeschnitten und appliziert. Dabei werden sie mit 0,1 bis 300 g Wirkstoff/ha (in der Regel 25%-ig formuliert) in wässriger Spritzbrühe besprüht. Die Wasseraufwandmenge beträgt ca. 200 l/ha.

4 Wochen nach der Applikation wird der Neuzuwachs im Gewicht ermittelt und in % zum Durchschnitt der unbehandelten Kontrollen dargestellt. Die nekrotischen Schäden sind in % zur gesamten Blattfläche aufgeführt.

Der Neuzuwachs der behandelten Pflanzen erweist sich als deutlich geringer als der der unbehandelten Kontrollen.

Beispiel B5: Wuchsregulierung an Sojabohnen

Versuchspflanzen der Sorte Williams werden in 11 cm-Tontöpfen mit Landerde (45 %), Torf (45 %) und Zonolit (10 %) angesät und in der Klimakammer bei einer Tagestemperatur von 24°C und einer Nachttemperatur von 19°C angezogen. Die Beleuchtungsdauer ist 16 Stunden pro Tage bei einer Intensität von ca. 350 Mikro-Einstein.

Ca. 24 Tage nach der Saat erfolgt das Umtopfen in 18 cm-Töpfe, 2 Pflanzen/Topf. Nach weiteren 12 Tagen und im Stadium von 5-6 Trifolia-Blätter findet die Applikation mit 0,1 bis 300 g Wirkstoff/ha statt, in der Regel 25%-ig formuliert und in wässriger Spritzbrühe. Die Wasseraufwandmenge beträgt ca. 200 l/ha.

Ca. 4 Wochen nach der Applikation findet die Auswertung statt. Hierbei wird die Höhe des Neuzuwachses gemessen und in % zum Durchschnitt der unbehandelten Kontrollen dargestellt. Die nekrotischen Schäden sind in % zur gesamten Blattfläche aufgeführt.

Die behandelten Pflanzen zeigen einen deutlich geringeren Neuzuwachs als die unbehandelten Kontrollen.

Beispiel B6: Wuchshemmung bei Getreide

Versuchspflanzen (Sommergerste der Sorte Iban) werden in 15 cm-Kunststofftöpfen mit steriler Landerde angesät und in der Klimakammer bei einer Tagestemperatur von 10-15 °C und einer Nachttemperatur von 5-10 °C angezogen. Die Beleuchtungsdauer ist 13.5 Stunden pro Tag bei einer Intensität von ca. 25000 Lux.

Ca. 34 Tage nach der Saat und dem Ausdünnen auf 4 Pflanzen/Topf erfolgt die Applikation mit 0,1 bis 300 g Wirkstoff/ha, in der Regel 25%-ig formuliert und in wässriger Spritzbrühe. Die Wasseraufwandmenge ist ca. 500 l/ha. Nach der Applikation werden die Pflanzen im Gewächshaus bei einer Tagestemperatur von mindestens 10 °C aufgestellt. Die Beleuchtungsdauer ist mind. 13.5 Stunden/Tag.

Ca. 28 Tage nach der Behandlung findet die Auswertung statt. Hierbei- wird die Höhe des Neuzuwachses in % zum Durchschnitt der unbehandelten Kontrollen dargestellt. Die nekrotischen Schäden sind in % zur gesamten Blattfläche aufgeführt.

Die behandelten Pflanzen weisen im Vergleich zu unbehandelten Kontrollen eine Verringerung des Neuzuwachses auf.

Beispiel B7: Wuchshemmung bei Gräsern

Eine Mischung von Gräsern (z.B. Poa, Festuca, Lolium, Bromus, Cynosurus) und Klee (Trifolium pratense/repens) wird in 15 cm-Kunststofftöpfen mit steriler Landerde angesät und im Gewächshaus bei einer Tagestemperatur von 21 °C und einer Nachttemperatur von 17 °C angezogen. Die Beleuchtungsdauer ist 13.5 Stunden/Tag bei einer Lichtintensität von mind. 7000 Lux. Nach dem Auflauf werden die Pflanzen wöchentlich auf ca. 6 cm Höhe zurückgeschnitten. Ca. 42 Tage nach der Saat und 1 Tag nach dem letzten Schnitt erfolgt die Applikation mit 0,1 bis 300 g Wirkstoff/ha, in der Regel 25%-ig formuliert und in wässriger Spritzbrühe. Die Wasseraufwand- menge ist ca. 500 l/ha.

Ca. 3 Wochen nach der Behandlung findet die Auswertung statt. Hierbei wird die Höhe des Neuzuwachses gemessen und in % zum Durchschnitt der unbehandelten Kontrollen dargestellt. Die nekrotischen Schäden sind in % zur gesamten Blattfläche aufgeführt.

Die geprüften Verbindungen der Tabelle 1 bewirken eine Reduzierung des Neuzuwachses im Vergleich zur unbehandelten Kontrolle.

**Patentansprüche**

**1.** Verbindungen der Formel I

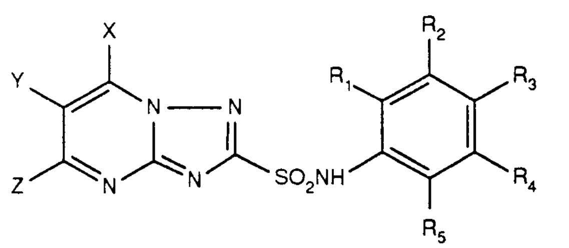

(I)

worin

$R_1$ für Halogen, Phenyl, O-Phenyl, $C_1$-$C_4$-Alkyl, $C_1$-$C_4$-Halogenalkyl, $-OR_8$, $-OR_9$, Nitro, Hydroxy, Cyano, $-SR_6$, $-SOR_6$, $-SO_2R_6$, $-SR_8$, $-SOR_8$, $-SO_2R_8$, $-SO_3R_8$, $-SR_9$, $SOR_9$, $-SO_2R_9$, $-SO_2NR_{10}(R_{10})$, $-SO_3R_9$, $-COOR_7$, $-CONHR_9$, $-CONR_9(R_9)$, Formyl,

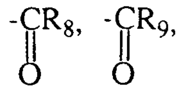

$-NH_2$, $NHR_9$ oder $-NR_9(R_9)$;
$R_2$ und $R_4$ unabhängig voneinander für Wasserstoff, Halogen, $C_1$-$C_4$-Alkyl, $-COOR_7$, oder $-OR_9$;
$R_3$ für Wasserstoff oder Halogen;
$R_5$ für Wasserstoff, Halogen, Phenyl, O-Phenyl, $C_1$-$C_4$-Alkyl, $C_1$-$C_4$-Halogenalkyl, $-OR_8$, $-OR_9$, Nitro, Hydroxy, Cyano, $SR_6$, $-SOR_6$, $-SO_2R_6$, $-SR_8$, $-SOR_8$, $-SO_3R_8$, $-SR_9$, $SOR_9$, $-SO_2R_9$, $-SO_2NR_{10}(R_{10})$ $-SO_3R_9$, $-COOR_7$, $-CONHR_9$, $-CONR_9(R_9)$, Formyl,

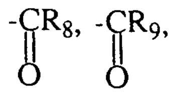

$-NH_2$, $NHR_9$ oder $-NR_9(R_9)$;
$R_6$ für Wasserstoff, Phenyl, Benzyl, $C_2$-$C_4$-Alkenyl, $C_2$-$C_4$-Alkinyl oder durch $-OR_9$ substituiertes $C_1$-$C_4$-Alkyl;
$R_7$ für $C_1$-$C_4$-Alkyl, $C_2$-$C_4$-Alkenyl, $C_2$-$C_4$-Alkinyl, Phenyl oder Benzyl;
$R_8$ für $C_1$-$C_4$-Halogenalkyl;
$R_9$ für $C_1$-$C_4$-Alkyl;
$R_{10}$ für Wasserstoff oder $C_1$-$C_4$-Alkyl;
X, Y, und Z unabhängig voneinander für Wasserstoff, $C_1$-$C_4$-Alkyl, $C_1$-$C_4$-Halogenalkyl, Phenylthio, Benzylthio, Hydroxy, Halogen, $-OR_8$, $-OR_9$, durch $-OR_9$ substituiertes $C_1$-$C_4$-Alkyl, durch $OR_8$ substituiertes $C_1$-$C_4$-Alkyl, $-NH_2$, $-NHR_9$, $-NR_9(R_9)$, $C_3$-$C_6$-Cycloalkyl, durch Halogen, $-OR_8$, $-OR_9$, $-SR_8$, $-SR_9$, oder $C_1$-$C_4$-Alkyl substituiertes $C_3$-$C_6$-Cycloalkyl oder X und Y zusammen oder Y und Z zusammen bilden eine $C_2$-$C_3$-Alkylenbrücke, die durch Sauerstoff oder Schwefel unterbrochen sein kann; stehen, mit der Massgabe, dass mindestens einer der Substituenten X, Y und Z für $C_3$-$C_6$-Cycloalkyl oder durch Halogen, $-OR_8$, $-OR_9$, $-SR_8$, $-SR_9$ oder $C_1$-$C_4$-Alkyl substituiertes $C_3$-$C_6$-Cycloalkyl steht; sowie die Salze dieser Verbindungen.

**2.** Verbindungen der Formel I gemäss Anspruch 1, dadurch gekennzeichnet, dass
$R_1$ für Halogen, Nitro, $C_1$-$C_4$-Alkyl, $C_1$-$C_4$-Halogenalkyl, $-OR_8$, $-OR_9$, Hydroxy, Cyano, oder $-COOR_7$;
$R_5$ für Wasserstoff, Halogen, Nitro, $C_1$-$C_4$-Alkyl, $C_1$-$C_4$-Halogenalkyl, $-OR_8$, $-OR_9$, Hydroxy, Cyano oder $-COOR_7$; und
X, Y und Z unabhängig voneinander für Wasserstoff, $C_1$-$C_4$-Alkyl, $C_1$-$C_4$-Halogenalkyl, durch $-OR_9$ substituiertes $C_1$-$C_4$-Alkyl, durch $-OR_8$ substituiertes $C_1$-$C_4$-Alkyl, Halogen, $C_3$-$C_6$-Cycloalkyl oder durch Halogen, $-OR_8$, $-OR_9$, $-SR_8$, $-SR_9$ oder $C_1$-$C_4$-Alkyl substituiertes $C_3$-$C_6$-Cycloalkyl stehen.

**3.** Verbindungen der Formel I gemäss Anspruch 1, dadurch gekennzeichnet, dass
$R_1$ für Halogen, Nitro, $C_1$-$C_2$-Alkyl, $C_1$-$C_2$-Halogenalkyl, $-OR_8$, $-OR_9$, Hydroxy, Cyano oder $-COOR_7$,
$R_2$ und $R_4$ unabhängig voneinander für Wasserstoff, Halogen, $C_1$-$C_2$-Alkyl, $-COOR_7$, oder $-OR_9$;
$R_5$ für Wasserstoff, Halogen, Nitro, $C_1$-$C_2$-Alkyl, $C_1$-$C_2$-Halogenalkyl, $-OR_8$, $-OR_9$, Hydroxy, Cyano, $-COOR_7$;
$R_7$ für $C_1$-$C_2$-Alkyl, $C_2$-$C_3$-Alkenyl, $C_2$-$C_3$-Alkinyl, Phenyl oder Benzyl;
$R_8$ für $C_1$-$C_2$-Halogenalkyl;
$R_9$ für $C_1$-$C_2$-Alkyl; und
X, Y und Z unabhängig voneinander für Wasserstoff, $C_1$-$C_2$-Alkyl, $C_1$-$C_2$-Halogenalkyl, Halogen, durch $-OR_9$ substituiertes $C_1$-$C_4$-Alkyl, durch $-OR_8$ substituiertes $C_1$-$C_4$-Alkyl, $C_3$-$C_6$-Cycloalkyl oder durch Halogen, $-OR_8$, $-OR_9$ oder $C_1$-$C_2$-Alkyl substituiertes $C_3$-$C_6$-Cycloalkyl stehen.

**4.** Verbindungen der Formel I gemäss Anspruch 3, dadurch gekennzeichnet, dass
$R_1$ für Fluor, Chlor, Methyl, Trifluormethyl oder Methoxy;
$R_2$ und $R_4$ unabhängig voneinander für Wasserstoff, Fluor, Chlor, Methyl oder Methoxy;
$R_3$ für Wasserstoff;
$R_5$ für Wasserstoff, Fluor, Chlor, Methyl, Trifluormethyl oder Methoxy; und

X, Y und Z unabhängig voneinander für Wasserstoff, Methyl, Cyclopropyl, 1-Methylcyclopropyl, 2-Fluorcyclopropyl, 2,3-Difluorcyclopropyl, 2-Chlor-cyclopropyl, 2,3-Dichlorcyclopropyl, 2-Methylcyclopropyl, 2,3-Dimethylcyclopropyl, Cyclopentyl, Fluor, Chlor, Trifluormethyl oder Methoxy stehen.

**5.** Verbindungen der Formel Ia

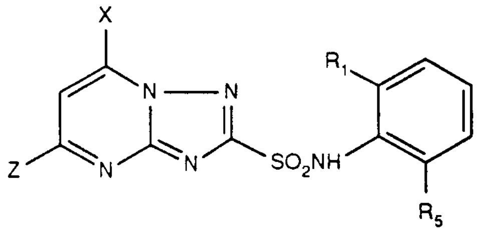

(Ia)

worin
in welcher
$R_1$ für Halogen, Phenyl, O-Phenyl, $C_1$-$C_4$-Alkyl, $C_1$-$C_4$-Halogenalkyl, $-OR_8$, $-OR_9$, Nitro, Hydroxy, Cyano, $-SR_6$, $-SOR_6$, $-SO_2R_6$, $-SR_8$, $-SOR_8$, $-SO_2R_8$, $-SO_3R_8$, $-SR_9$, $SOR_9$, $-SO_2R_9$, $-SO_2NR_{10}(R_{10})$, $-SO_3R_9$, $-COOR_7$, $-CONHR_9$, $-CONR_9(R_9)$, Formyl,

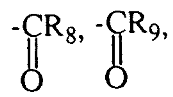

$-NH_2$, $NHR_9$ oder $-NR_9(R_9)$;
$R_5$ für Wasserstoff, Halogen, Phenyl, O-Phenyl, $C_1$-$C_4$-Alkyl, $C_1$-$C_4$-Halogenalkyl, $-OR_8$, $-OR_9$, Nitro, Hydroxy, Cyano, $SR_6$, $-SOR_6$, $-SO_2R_6$, $-SR_8$, $-SOR_8$, $-SO_3R_8$, $-SR_9$, $SOR_9$, $-SO_2R_9$, $-SO_2NR_{10}(R_{10})$, $-SO_3R_9$, $-COOR_7$, $-CONHR_9$, $-CONR_9(R_9)$, Formyl,

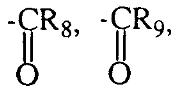

$-NH_2$, $NHR_9$ oder $-NR_9(R_9)$;
$R_6$ für Wasserstoff, Phenyl, Benzyl, $C_2$-$C_4$-Alkenyl, $C_2$-$C_4$-Alkinyl oder durch $-OR_9$ substituiertes $C_1$-$C_4$-Alkyl;
$R_7$ für $C_1$-$C_4$-Alkyl, $C_2$-$C_4$-Alkenyl, $C_2$-$C_4$-Alkinyl, Phenyl oder Benzyl;
$R_8$ für $C_1$-$C_4$-Halogenalkyl;
$R_9$ für $C_1$-$C_4$-Alkyl;
$R_{10}$ für Wasserstoff oder $C_1$-$C_4$-Alkyl;
X und Z unabhängig voneinander für Wasserstoff, $C_1$-$C_4$-Alkyl, $C_1$-$C_4$-Halogenalkyl, Phenylthio, Benzylthio, Hydroxy, Halogen, $-OR_8$, $-OR_9$, durch $-OR_9$ substituiertes $C_1$-$C_4$-Alkyl, durch $-OR_8$ substituiertes $C_1$-$C_4$-Alkyl, $-NH_2$, $-NHR_9$, $-NR_9(R_9)$, $C_3$-$C_6$-Cycloalkyl oder durch Halogen, $-OR_8$, $-OR_9$, $-SR_8$, $-SR_9$, oder $C_1$-$C_4$-Alkyl substituiertes $C_3$-$C_6$-Cycloalkyl stehen,
mit der Massgabe, dass mindestens einer der Substituenten X oder Z für $C_3$-$C_6$-Cycloalkyl oder durch Halogen, $-OR_8$, $-OR_9$, $-SR_8$, $-SR_9$ oder $C_1$-$C_4$-Alkyl substituiertes $C_3$-$C_6$-Cycloalkyl steht; sowie die Salze dieser Verbindungen.

**6.** Verbindungen der Formel Ia gemäss Anspruch 5, dadurch gekennzeichnet, dass
$R_1$ für Halogen, Nitro, $C_1$-$C_4$-Alkyl, $C_1$-$C_4$-Halogenalkyl, $-OR_8$, $-OR_9$, Hydroxy, Cyano, oder $-COOR_7$;
$R_5$ für Wasserstoff, Halogen, Nitro, $C_1$-$C_4$-Alkyl, $C_1$-$C_4$-Halogenalkyl, $-OR_8$, $-OR_9$, Hydroxy, Cyano oder $-COOR_7$; und
X und Z unabhängig voneinander für Wasserstoff, $C_1$-$C_4$-Alkyl, $C_1$-$C_4$-Halogenalkyl, durch $-OR_9$ substituiertes $C_1$-$C_4$-Alkyl, durch $-OR_8$ substituiertes $C_1$-$C_4$-Alkyl, Halogen, $C_3$-$C_6$-Cycloalkyl oder durch

Halogen, $-OR_8$, $-OR_9$, $-SR_8$, $-SR_9$ oder $C_1$-$C_4$-Alkyl substituiertes $C_3$-$C_6$-Cycloalkyl stehen.

**7.** Verbindungen der Formel Ia gemäss Anspruch 5, dadurch gekennzeichnet, dass
$R_1$ für Halogen, Nitro, $C_1$-$C_2$-Alkyl, $C_1$-$C_2$-Halogenalkyl, $-OR_8$, $-OR_9$, Hydroxy, Cyano oder $-COOR_7$,
$R_5$ für Wasserstoff, Halogen, Nitro, $C_1$-$C_2$-Alkyl, $C_1$-$C_2$-Halogenalkyl, $-OR_8$, $-OR_9$, Hydroxy, Cyano, $-COOR_7$;
$R_7$ für $C_1$-$C_2$-Alkyl, $C_2$-$C_3$-Alkenyl, $C_2$-$C_3$-Alkinyl, Phenyl oder Benzyl;
$R_8$ für $C_1$-$C_2$-Halogenalkyl;
$R_9$ für $C_1$-$C_2$-Alkyl; und
X und Z unabhängig voneinander für Wasserstoff, $C_1$-$C_2$-Alkyl, $C_1$-$C_2$-Halogenalkyl, Halogen, $C_3$-$C_6$-Cycloalkyl oder durch Halogen, $-OR_8$, $-OR_9$ oder $C_1$-$C_2$-Alkyl substituiertes $C_3$-$C_6$-Cycloalkyl stehen.

**8.** Verbindungen der Formel Ia gemäss Anspruch 5, dadurch gekennzeichnet, dass
$R_1$ für Fluor, Chlor, Methyl, Trifluormethyl oder Methoxy;
$R_5$ für Wasserstoff, Fluor, Chlor, Methyl, Trifluormethyl oder Methoxy; und
X und Z unabhängig voneinander für Wasserstoff, Methyl, Cyclopropyl, 1-Methylcyclopropyl, 2-Fluorcyclopropyl, 2,3-Difluorcyclopropyl, 2-Chlorcyclopropyl, 2,3-Dichlorcyclopropyl, 2-Methylcyclopropyl, 2,3-Dimethylcyclopropyl, Cyclopentyl, Fluor, Chlor, oder Methoxy stehen.

**9.** Verbindungen der Formel Ia gemäss Anspruch 5, dadurch gekennzeichnet, dass
$R_1$ für Fluor, Chlor, Methyl oder Trifluormethyl;
$R_5$ für Wasserstoff, Chlor, Methyl, oder Trifluormethyl; und
X und Z unabhängig voneinander für Wasserstoff, Cyclopropyl, 1-Methylcyclopropyl, 2-Fluorcyclopropyl, 2,3-Difluorcyclopropyl, 2-Chlorcyclopropyl, 2,3-Dichlorcyclopropyl, 2-Methylcyclopropyl, 2,3-Dimethylcyclopropyl, Methyl, Methoxy, Fluor oder Chlor stehen.

**10.** Verbindungen der Formel Ia gemäss Anspruch 9, dadurch gekennzeichnet, dass X für Cyclopropyl steht.

**11.** Verbindungen der Formel I gemäss Anspruch 1, dadurch gekennzeichnet, dass X für $C_3$-$C_6$-Cycloalkyl oder durch Halogen, $-OR_8$, $-OR_9$, $-SR_8$, $-SR_9$ oder $C_1$-$C_4$-Alkyl subtituiertes $C_3$-$C_6$-Cycloalkyl steht.

**12.** Verbindungen der Formel I gemäss Anspruch 1, dadurch gekennzeichnet, dass X für Cyclopropyl, 1-Methylcyclopropyl, 2-Fluorcyclopropyl, 2,3-Difluorcyclopropyl, 2-Chlorcyclopropyl, 2,3-Dichlorcyclopropyl, 2-Methylcyclopropyl oder 2,3-Dimethylcyclopropyl steht.

**13.** Verbindungen der Formel I gemäss Anspruch 1, dadurch gekennzeichnet, dass X für Cyclopropyl steht.

**14.** 5-Methyl-7-cyclopropyl-N-(2,6-dichlorphenyl)-1,2,4-triazolo[1,5-a]pyrimidin-2-sulfonamid gemäss Anspruch 1.

**15.** Verfahren zur Herstellung der Verbindungen der Formel I gemäss Anspruch 1, dadurch gekennzeichnet, dass man ein primäres Amin der Formel II

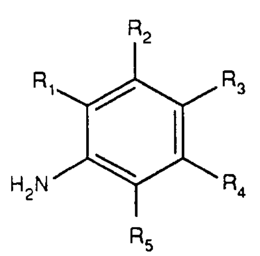

(II)

worin $R_1$, $R_2$, $R_3$, $R_4$ und $R_5$ die unter Formel I gegebene Bedeutung haben, in Gegenwart einer Base mit einem Triazolopyrimidinylsulfonylchlorid der Formel III

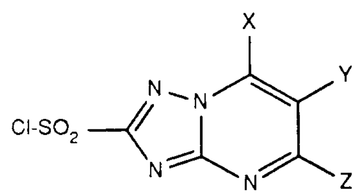

(III)

worin X, Y und Z die unter Formel I gegebenen Bedeutungen haben, umsetzt.

**16.** Ein herbizides und den Pflanzenwuchs hemmendes Mittel, dadurch gekennzeichnet, dass es neben Träger- und/oder anderen Zuschlagstoffen als Wirkstoff mindestens ein Triazolylsulfonamid der Formel I, gemäss Anspruch 1, enthält.

**17.** Mittel gemäss Anspruch 16, dadurch gekennzeichnet, dass es zwischen 0,1 % und 95 % Wirkstoff der Formel I gemäss Anspruch 1 enthält.

**18.** Verfahren zur Bekämpfung unerwünschten Pflanzenwachstums, dadurch gekennzeichnet, dass man einen Wirkstoff der Formel I, gemäss Anspruch 1, oder ein diesen Wirkstoff enthaltendes Mittel in einer wirksamen Menge auf die Pflanzen oder deren Lebensraum appliziert.

**19.** Verfahren gemäss Anspruch 18, dadurch gekennzeichnet, dass man eine Wirkstoffmenge zwischen 0,001 und 5 kg pro Hektar appliziert.

**20.** Verfahren zur Hemmung des Pflanzenwachstums, dadurch gekennzeichnet, dass man einen Wirkstoff der Formel I, gemäss Anspruch 1, oder ein diesen Wirkstoff enthaltendes Mittel in einer wirksamen Menge auf die Pflanzen oder deren Lebensraum appliziert.

**21.** Verfahren zur Beeinflussung des Pflanzenwachstums zum Zweck der Ertragssteigerung, dadurch gekennzeichnet, dass man einen Wirkstoff der Formel I, gemäss Anspruch 1, oder ein diesen Wirkstoff enthaltendes Mittel in einer wirksamen Menge auf die Pflanzen oder deren Lebensraum appliziert.

**22.** Verfahren gemäss Anspruch 18, zur selektiven pre- oder post-emergenten Bekämpfung von Unkräutern in Nutzpflanzenkulturen.

**23.** Triazolopyrimidinylsulfonylchloride der Formel III

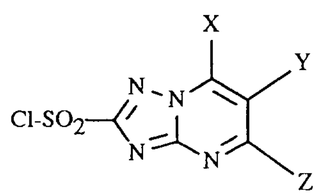

(III),

worin
X, Y und Z unabhängig voneinander Wasserstoff, $C_1$-$C_4$-Alkyl, $C_1$-$C_4$-Halogenalkyl, Phenylthio, Benzylthio, Hydroxy, Halogen, -$OR_8$, -$OR_9$, durch -$OR_9$ substituiertes $C_1$-$C_4$-Alkyl, durch -$OR_8$ substituiertes $C_1$-$C_4$-Alkyl, -$NH_2$, -$NHR_9$, -$NR_9(R_9)$, $C_3$-$C_6$-Cycloalkyl, durch Halogen, -$OR_8$, -$OR_9$, -$SR_8$, -$SR_9$, oder $C_1$-$C_4$-Alkyl substituiertes $C_3$-$C_6$-Cycloalkyl sind, oder X und Y zusammen oder Y und Z zusammen eine $C_2$-$C_3$-Alkylenbrücke bilden, die durch Sauerstoff oder Schwefel unterbrochen sein kann, mit den Massgaben, dass (1) mindestens einer der Substituenten X, Y und Z für $C_3$-$C_6$-Cycloalkyl oder durch Halogen, -$OR_8$, -$OR_9$, -$SR_8$, -$SR_9$, oder $C_1$-$C_4$-Alkyl substituiertes $C_3$-$C_6$-Cycloalkyl steht, und (2) Y verschieden von Wasserstoff ist, wenn X und Z unabhängig voneinander Wasserstoff, $C_1$-$C_3$-Alkyl, Trifluormethyl oder Cyclopropyl sind.

**24.** Verbindungen der Formel V

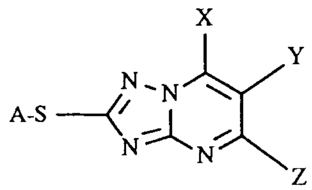

(V),

worin
X, Y und Z unabhängig voneinander Wasserstoff, $C_1$-$C_4$-Alkyl, $C_1$-$C_4$-Halogenalkyl, Phenylthio, Benzylthio, Hydroxy, Halogen, $-OR_8$, $-OR_9$, durch $-OR_9$ substituiertes $C_1$-$C_4$-Alkyl, durch $-OR_8$ substituiertes $C_1$-$C_4$-Alkyl, $-NH_2$, $-NHR_9$, $-NR_9(R_9)$, $C_3$-$C_6$-Cycloalkyl, durch Halogen, $-OR_8$, $-OR_9$, $-SR_8$, $-SR_9$, oder $C_1$-$C_4$-Alkyl substituiertes $C_3$-$C_6$-Cycloalkyl sind, oder X und Y zusammen oder Y und Z zusammen eine $C_2$-$C_3$-Alkylenbrücke bilden, die durch Sauerstoff oder Schwefel unterbrochen sein kann; und A Wasserstoff, Isopropyl oder Benzyl bedeutet, mit den Massgaben, dass (1) mindestens einer der Substituenten X, Y und Z für $C_3$-$C_6$-Cycloalkyl oder durch Halogen, $-OR_8$, $-OR_9$, $-SR_8$, $-SR_9$, oder $C_1$-$C_4$-Alkyl substituiertes $C_3$-$C_6$-Cycloalkyl steht, und (2) Y verschieden von Wasserstoff ist, wenn X und Z unabhängig voneinander Wasserstoff, $C_1$-$C_3$-Alkyl, Trifluormethyl oder Cyclopropyl sind.

## Claims

**1.** A compound of formula I

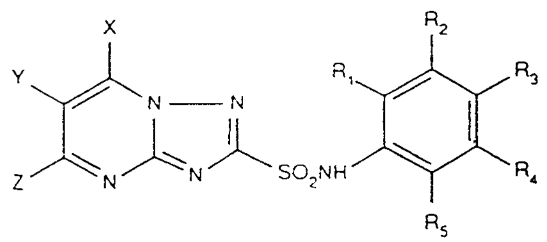

(I)

wherein
$R_1$ is halogen, phenyl, O-phenyl, $C_1$-$C_4$alkyl, $C_1$-$C_4$haloalkyl, $-OR_8$, $-OR_9$, nitro, hydroxy, cyano, $-SR_6$, $-SOR_6$, $-SO_2R_6$, $-SR_8$, $-SOR_8$, $-SO_2R_8$, $-SO_3R_8$, $-SR_9$, $-SOR_9$, $-SO_2R_9$, $-SO_2NR_{10}(R_{10})$, $-SO_3R_9$, $-COOR_7$, $-CONHR_9$, $-CONR_9(R_9)$,formyl,

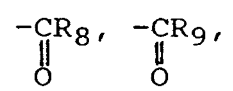

$-NH_2$, $-NHR_9$ or $-NR_9(R_9)$;
$R_2$ and $R_4$ independently of one another are hydrogen, halogen, $C_1$-$C_4$alkyl, $-COOR_7$ or $-OR_9$;
$R_3$ is hydrogen or halogen;
$R_5$ is hydrogen, halogen, phenyl, O-phenyl, $C_1$-$C_4$alkyl, $C_1$-$C_4$haloalkyl, $-OR_8$, $-OR_9$, nitro, hydroxy, cyano, $-SR_6$, $-SOR_6$, $-SO_2R_6$, $-SR_8$, $-SOR_8$, $-SO_3R_8$, $-SR_9$, $-SOR_9$, $-SO_2R_9$, $-SO_2NR_{10}(R_{10})$, $-SO_3R_9$, $-COOR_7$, $-CONHR_9$, $-CONR_9(R_9)$,formyl,

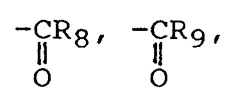

$-NH_2$, $-NHR_9$ or $-NR_9(R_9)$;
$R_6$ is hydrogen, phenyl, benzyl, $C_2$-$C_4$alkenyl, $C_2$-$C_4$alkynyl, or $C_1$-$C_4$alkyl substituted by $-OR_9$;
$R_7$ is $C_1$-$C_4$alkyl, $C_2$-$C_4$alkenyl, $C_2$-$C_4$alkynyl, phenyl or benzyl;

$R_8$ is $C_1$-$C_4$haloalkyl;
$R_9$ is $C_1$-$C_4$alkyl;
$R_{10}$ is hydrogen or $C_1$-$C_4$alkyl;
X, Y and Z independently of one another are hydrogen, $C_1$-$C_4$alkyl, $C_1$-$C_4$haloalkyl, phenylthio, benzylthio, hydroxy, halogen, $-OR_8$, $-OR_9$, $C_1$-$C_4$alkyl substituted by $-OR_9$, $C_1$-$C_4$alkyl substituted by $-OR_8$, or $-NH_2$, $-NHR_9$, $-NR_9(R_9)$, $C_3$-$C_6$cycloalkyl, $C_3$-$C_6$cycloalkyl substituted by halogen, $-OR_8$, $-OR_9$, $-SR_8$, $-SR_9$ or by $C_1$-$C_4$alkyl, or X and Y together or Y and Z together form a $C_2$-$C_3$alkylene bridge which may be interrupted by oxygen or sulfur; with the proviso that at least one of the substituents X, Y and Z is $C_3$-$C_6$cycloalkyl or $C_3$-$C_6$cycloalkyl substituted by halogen, $-OR_8$, $-OR_9$, $-SR_8$, $-SR_9$ or by $C_1$-$C_4$alkyl; or a salt of such a compound.

**2.** A compound of formula I according to claim 1, wherein
$R_1$ is halogen, nitro, $C_1$-$C_4$alkyl, $C_1$-$C_4$haloalkyl, $-OR_8$, $-OR_9$, hydroxy, cyano or $-COOR_7$;
$R_5$ is hydrogen, halogen, nitro, $C_1$-$C_4$alkyl, $C_1$-$C_4$haloalkyl, $-OR_8$, $-OR_9$, hydroxy, cyano or $-COOR_7$; and
X, Y and Z independently of one another are hydrogen, $C_1$-$C_4$alkyl, $C_1$-$C_4$haloalkyl, $C_1$-$C_4$alkyl substituted by $-OR_9$, $C_1$-$C_4$alkyl substituted by $-OR_8$, or halogen, $C_3$-$C_6$cycloalkyl or $C_3$-$C_6$cycloalkyl substituted by halogen, $-OR_8$, $-OR_9$, $-SR_8$, $-SR_9$ or by $C_1$-$C_4$alkyl.

**3.** A compound of formula I according to claim 1, wherein
$R_1$ is halogen, nitro, $C_1$-$C_2$alkyl, $C_1$-$C_2$haloalkyl, $-OR_8$, $-OR_9$, hydroxy, cyano or $-COOR_7$;
$R_2$ and $R_4$ independently of one another are hydrogen, halogen, $C_1$-$C_2$alkyl, $-COOR_7$ or $-OR_9$;
$R_5$ is hydrogen, halogen, nitro, $C_1$-$C_2$alkyl, $C_1$-$C_2$haloalkyl, $-OR_8$, $-OR_9$, hydroxy, cyano or $-COOR_7$;
$R_7$ is $C_1$-$C_2$alkyl, $C_2$-$C_3$alkenyl, $C_2$-$C_3$alkynyl, phenyl or benzyl;
$R_8$ is $C_1$-$C_2$haloalkyl;
$R_9$ is $C_1$-$C_2$alkyl; and
X, Y and Z independently of one another are hydrogen, $C_1$-$C_2$alkyl, $C_1$-$C_2$haloalkyl, halogen, $C_1$-$C_4$alkyl substituted by $-OR_9$, $C_1$-$C_4$alkyl substituted by $-OR_8$, $C_3$-$C_6$cycloalkyl or $C_3$-$C_6$cycloalkyl substituted by halogen, $-OR_8$, $-OR_9$ or by $C_1$-$C_2$alkyl.

**4.** A compound of formula I according to claim 3, wherein
$R_1$ is fluorine, chlorine, methyl, trifluoromethyl or methoxy;
$R_2$ and $R_4$ independently of one another are hydrogen, fluorine, chlorine, methyl or methoxy;
$R_3$ is hydrogen;
$R_5$ is hydrogen, fluorine, chlorine, methyl, trifluoromethyl or methoxy; and
X, Y and Z independently of one another are hydrogen, methyl, cyclopropyl, 1-methylcyclopropyl, 2-fluorocyclopropyl, 2,3-difluorocyclopropyl, 2-chlorocyclopropyl, 2,3-dichlorocyclopropyl, 2-methylcyclopropyl, 2,3-dimethylcyclopropyl, cyclopentyl, fluorine, chlorine, trifluoromethyl or methoxy.

**5.** A compound of formula Ia

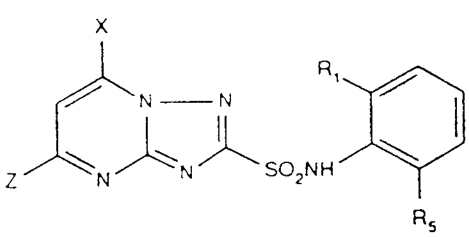

(Ia)

wherein
$R_1$ is halogen, phenyl, O-phenyl, $C_1$-$C_4$alkyl, $C_1$-$C_4$haloalkyl, $-OR_8$, $-OR_9$, nitro, hydroxy, cyano, $-SR_6$, $-SOR_6$, $-SO_2R_6$, $-SR_8$, $-SOR_8$, $-SO_2R_8$, $-SO_3R_8$, $-SR_9$, $-SOR_9$, $-SO_2R_9$, $-SO_2NR_{10}(R_{10})$, $-SO_3R_9$, $-COOR_7$, $-CONHR_9$, $-CONR_9(R_9)$,formyl,

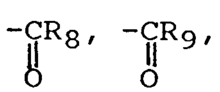

$-NH_2$, $-NHR_9$ or $-NR_9(R_9)$;
$R_5$ is hydrogen, halogen, phenyl, O-phenyl, $C_1$-$C_4$alkyl, $C_1$-$C_4$haloalkyl, $-OR_8$, $-OR_9$, nitro, hydroxy, cyano, $-SR_6$, $-SOR_6$, $-SO_2R_6$, $-SR_8$, $-SOR_8$, $-SO_3R_8$, $-SR_9$, $-SOR_9$, $-SO_2R_9$, $-SO_2NR_{10}(R_{10})$, $-SO_3R_9$, $-COOR_7$, $-CONHR_9$, $-CONR_9(R_9)$,formyl,

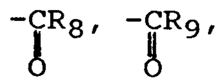

$-NH_2$, $-NHR_9$ or $-NR_9(R_9)$;
$R_6$ is hydrogen, phenyl, benzyl, $C_2$-$C_4$alkenyl, $C_2$-$C_4$alkynyl, or $C_1$-$C_4$alkyl substituted by $-OR_9$;
$R_7$ is $C_1$-$C_4$alkyl, $C_2$-$C_4$alkenyl, $C_2$-$C_4$alkynyl, phenyl or benzyl;
$R_8$ is $C_1$-$C_4$haloalkyl;
$R_9$ is $C_1$-$C_4$alkyl;
$R_{10}$ is hydrogen or $C_1$-$C_4$alkyl;
X and Z independently of one another are hydrogen, $C_1$-$C_4$alkyl, $C_1$-$C_4$haloalkyl, phenylthio, benzylthio, hydroxy, halogen, $-OR_8$, $-OR_9$, $C_1$-$C_4$alkyl substituted by $-OR_9$, $C_1$-$C_4$alkyl substituted by $-OR_8$, or $-NH_2$, $-NHR_9$, $-NR_9(R_9)$, $C_3$-$C_6$cycloalkyl, or $C_3$-$C_6$cycloalkyl substituted by halogen, $-OR_8$, $-OR_9$, $-SR_8$, $-SR_9$ or by $C_1$-$C_4$alkyl, with the proviso that at least one of the substituents X and Z is $C_3$-$C_6$cycloalkyl or $C_3$-$C_6$cycloalkyl substituted by halogen, $-OR_8$, $-OR_9$, $-SR_8$, $-SR_9$ or by $C_1$-$C_4$alkyl; or a salt of such a compound.

**6.** A compound of formula Ia according to claim 5, wherein
$R_1$ is halogen, nitro, $C_1$-$C_4$alkyl, $C_1$-$C_4$haloalkyl, $-OR_8$, $-OR_9$, hydroxy, cyano or $-COOR_7$;
$R_5$ is hydrogen, halogen, nitro, $C_1$-$C_4$alkyl, $C_1$-$C_4$haloalkyl, $-OR_8$, $-OR_9$, hydroxy, cyano or $-COOR_7$; and
X and Z independently of one another are hydrogen, $C_1$-$C_4$alkyl, $C_1$-$C_4$haloalkyl, $C_1$-$C_4$alkyl substituted by $-OR_9$, $C_1$-$C_4$alkyl substituted by $-OR_8$, or halogen, $C_3$-$C_6$cycloalkyl or $C_3$-$C_6$cycloalkyl substituted by halogen, $-OR_8$, $-OR_9$, $-SR_8$, $-SR_9$ or by $C_1$-$C_4$alkyl.

**7.** A compound of formula Ia according to claim 5, wherein
$R_1$ is halogen, nitro, $C_1$-$C_2$alkyl, $C_1$-$C_2$haloalkyl, $-OR_8$, $-OR_9$, hydroxy, cyano or $-COOR_7$;
$R_5$ is hydrogen, halogen, nitro, $C_1$-$C_2$alkyl, $C_1$-$C_2$haloalkyl, $-OR_8$, $-OR_9$, hydroxy, cyano or $-COOR_7$;
$R_7$ is $C_1$-$C_2$alkyl, $C_2$-$C_3$alkenyl, $C_2$-$C_3$alkynyl, phenyl or benzyl;
$R_8$ is $C_1$-$C_2$haloalkyl;
$R_9$ is $C_1$-$C_2$alkyl; and
X and Z independently of one another are hydrogen, $C_1$-$C_2$alkyl, $C_1$-$C_2$haloalkyl, halogen, $C_3$-$C_6$cycloalkyl or $C_3$-$C_6$cycloalkyl substituted by halogen, $-OR_8$, $-OR_9$ or by $C_1$-$C_2$alkyl.

**8.** A compound of formula Ia according to claim 5, wherein
$R_1$ is fluorine, chlorine, methyl, trifluoromethyl or methoxy;
$R_5$ is hydrogen, fluorine, chlorine, methyl, trifluoromethyl or methoxy; and
X and Z independently of one another are hydrogen, methyl, cyclopropyl, 1-methylcyclopropyl, 2-fluorocyclopropyl, 2,3-difluorocyclopropyl, 2-chlorocyclopropyl, 2,3-dichlorocyclopropyl, 2-methylcyclopropyl, 2,3-dimethylcyclopropyl, cyclopentyl, fluorine, chlorine or methoxy.

**9.** A compound of formula Ia according to claim 5, wherein
$R_1$ is fluorine, chlorine, methyl or trifluoromethyl;
$R_5$ is hydrogen, chlorine, methyl or trifluoromethyl; and
X and Z independently of one another are hydrogen, cyclopropyl, 1-methylcyclopropyl, 2-fluorocyclopropyl, 2,3-difluorocyclopropyl, 2-chlorocyclopropyl, 2,3-dichlorocyclopropyl, 2-methylcyclopropyl, 2,3-dimethylcyclopropyl, methyl, methoxy, fluorine or chlorine.

**10.** A compound of formula Ia according to claim 9, wherein X is cyclopropyl.

**11.** A compound of formula I according to claim 1, wherein X is $C_3$-$C_6$cycloalkyl or $C_3$-$C_6$cycloalkyl substituted by halogen, $-OR_8$, $-OR_9$, $-SR_8$, $-SR_9$ or by $C_1$-$C_4$alkyl.

**12.** A compound of formula I according to claim 1, wherein X is cyclopropyl, 1-methylcyclopropyl, 2-fluorocyclopropyl, 2,3-difluorocyclopropyl, 2-chlorocyclopropyl, 2,3-dichlorocyclopropyl, 2-methylcyclopropyl or 2,3-dimethylcyclopropyl.

**13.** A compound of formula I according to claim 1, wherein X is cyclopropyl.

**14.** 5-Methyl-7-cyclopropyl-N-(2,6-dichlorophenyl)-1,2,4triazolo[1,5-a]pyrimidine-2-sulfonamide according to claim 1.

**15.** A process for the preparation of a compound of formula I according to claim 1, which process comprises reacting a primary amine of formula II

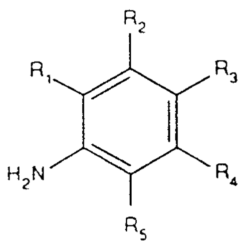

(II),

wherein $R_1$, $R_2$, $R_3$, $R_4$ and $R_5$ are as defined under formula I, in the presence of a base, with a triazolopyrimidinylsulfonyl chloride of formula III

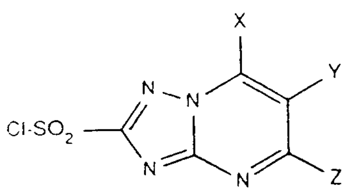

(III),

wherein X, Y and Z are as defined under formula I.

**16.** A herbicidal and plant growth inhibiting composition, which comprises as active ingredient at least one triazolylsulfonamide of formula I according to claim 1, together with carriers and/or other adjuvants.

**17.** A composition according to claim 16, which comprises from 0.1 % to 95 % of a compound of formula I according to claim 1.

**18.** A method of controlling undesired plant growth, which comprises applying to the plants or to the locus thereof an effective amount of a compound of formula I according to claim 1 or of a composition comprising that compound.

**19.** A method according to claim 18, wherein an amount of from 0.001 to 5 kg of compound is applied per hectare.

**20.** A method of inhibiting plant growth, which comprises applying to the plants or to the locus thereof an effective amount of a compound of formula I according to claim 1 or of a composition comprising that compound.

**21.** A method of influencing plant growth for the purpose of increasing yield, which method comprises applying to the plants or to the locus thereof an effective amount of a compound of formula I according to claim 1 or of a composition comprising that compound.

**22.** A method according to claim 18 of selectively controlling weeds pre- or post-emergence in crops of useful plants.

**23.** A triazolopyrimidinylsulfonyl chloride of formula III

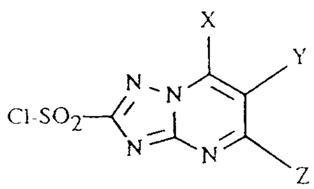

(III)

wherein
X, Y and Z independently of one another are hydrogen, $C_1$-$C_4$alkyl, $C_1$-$C_4$haloalkyl, phenylthio, benzylthio, hydroxy, halogen, -$OR_8$, -$OR_9$, $C_1$-$C_4$alkyl substituted by -$OR_9$, $C_1$-$C_4$alkyl substituted by -$OR_8$, or -$NH_2$, -$NHR_9$, -$NR_9(R_9)$, $C_3$-$C_6$cycloalkyl, $C_3$-$C_6$cycloalkyl substituted by halogen, -$OR_8$, -$OR_9$, -$SR_8$, -$SR_9$ or by $C_1$-$C_4$alkyl, or X and Y together or Y and Z together form a $C_2$-$C_3$alkylene bridge which may be interruped by oxygen or sulfur; with the provisos (1) that at least one of the substituents X, Y and Z is $C_3$-$C_6$cycloalkyl or $C_3$-$C_6$cycloalkyl substituted by halogen, -$OR_8$, -$OR_9$, -$SR_8$, -$SR_9$ or by $C_1$-$C_4$alkyl, and (2) that Y is other than hydrogen when X and Z independently of one another are hydrogen, $C_1$-$C_3$alkyl, trifluoromethyl or cyclopropyl.

**24.** A compound of formula V

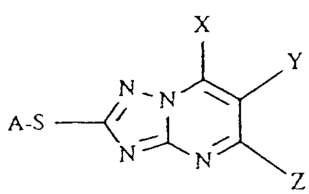

(V)

wherein
X, Y and Z independently of one another are hydrogen, $C_1$-$C_4$alkyl, $C_1$-$C_4$haloalkyl, phenylthio, benzylthio, hydroxy, halogen, -$OR_8$, -$OR_9$, $C_1$-$C_4$alkyl substituted by -$OR_9$, $C_1$-$C_4$alkyl substituted by -$OR_8$, or -$NH_2$, -$NHR_9$, -$NR_9(R_9)$, $C_3$-$C_6$cycloalkyl, $C_3$-$C_6$cycloalkyl substituted by halogen, -$OR_8$, -$OR_9$, -$SR_8$, -$SR_9$ or by $C_1$-$C_4$alkyl, or X and Y together or Y and Z together form a $C_2$-$C_3$alkylene bridge which may be interruped by oxygen or sulfur; and
A is hydrogen, isopropyl or benzyl;
with the provisos (1) that at least one of the substituents X, Y and Z is $C_3$-$C_6$cycloalkyl or $C_3$-$C_6$cycloalkyl substituted by halogen, -$OR_8$, -$OR_9$, $SR_8$, -$SR_9$ or by $C_1$-$C_4$alkyl, and (2) that Y is other than hydrogen when X and Z independently of one another are hydrogen, $C_1$-$C_3$alkyl, trifluoromethyl or cyclopropyl.

## Revendications

**1.** Composés de formule I

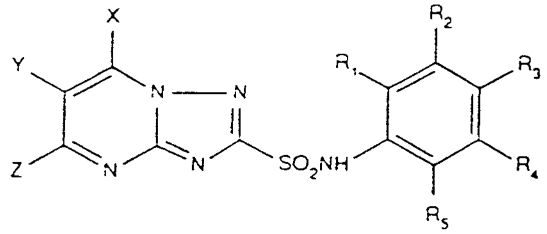

(I)

dans laquelle
$R_1$ représente un halogène, un groupe phényle, O-phényle, alkyle en C1-C4, halogénoalkyle en C1-C4,

$-OR_8$, $-OR_9$, nitro, hydroxy, cyano, $-SR_6$, $-SOR_6$, $-SO_2R_6$, $-SR_8$, $-SOR_8$, $-SO_2R_8$, $-SR_9$, $SOR_9$, $-SO_2R_9$, $-SO_2NR_{10}(R_{10})$, $-SO_3R_9$, $-COOR_7$, $-CONHR_9$, $-CONR_9(R_9)$, formyle,

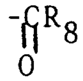, 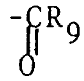,

$-NH_2$, $NHR_9$ ou $-NR_9(R_9)$;
$R_2$ et $R_4$ représentent chacun, indépendamment l'un de l'autre, l'hydrogène, un halogène, un groupe alkyle en C1-C4, $-COOR_7$ ou $-OR_9$;
$R_3$ représente l'hydrogène ou un halogène;
$R_5$ représente l'hydrogène, un halogène, un groupe phényle, O-phényle, alkyle en C1-C4, halogénoalkyle en C1-C4, $-OR_8$, $-OR_9$, nitro, hydroxy, cyano, $-SR_6$, $-SOR_6$, $-SO_2R_6$, $-SR_8$, $-SOR_8$, $-SO_3R_8$, $-SR_9$, $SOR_9$, $-SO_2R_9$, $-SO_2NR_{10}(R_{10})$, $-SO_3R_9$, $-COOR_7$, $-CONHR_9$, $-CONR_9(R_9)$,formyle,

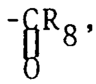, 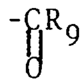,

$-NH_2$, $NHR_9$ ou $-NR_9(R_9)$;
$R_6$ représente l'hydrogène, un groupe phényle, benzyle, alcényle en C2-C4, alcynyle en C2-C4 ou un groupe alkyle en C1-C4 portant un substituant $-OR_9$;
$R_7$ représente un groupe alkyle en C1-C4, alcényle en C2-C4, alcynyle en C2-C4, phényle ou benzyle;
$R_8$ représente un groupe halogénoalkyle en C1-C4;
$R_9$ représente un groupe alkyle en C1-C4;
$R_{10}$ représente l'hydrogène ou un groupe alkyle en C1-C4; X, Y et Z représentent chacun, indépendamment les uns des autres, l'hydrogène, un groupe alkyle en C1-C4, halogénoalkyle en C1-C4, phénylthio, benzylthio, hydroxy, un halogène, un groupe $-OR_8$, $-OR_9$, un groupe alkyle en C1-C4 portant un substituant $-OR_9$, un groupe alkyle en C1-C4 portant un substituant $-OR_8$, un groupe $-NH_2$, $-NHR_9$, $-NR_9(R_9)$, un groupe cycloalkyle en C3-C6, un groupe cycloalkyle en C3-C6 substitué par des halogènes, des groupes $-OR_8$, $-OR_9$, $-SR_8$, $-SR_9$ ou alkyle en C1-C4, ou bien X et Y, ensemble, ou Y et Z, ensemble, forment un pont alkylène en C2-C3 qui peut être interrompu par l'oxygène ou le soufre; sous réserve que l'un au moins des symboles X, Y et Z représente un groupe cycloalkyle en C3-C6 ou un groupe cycloalkyle en C3-C6 substitué par des halogènes, des groupes $-OR_8$, $-OR_9$, $-SR_8$, $-SR_9$ ou alkyle en C1-C4, ainsi que les sels de ces composés.

**2.** Composés de formule I selon revendication 1, caractérisé s en ce que
$R_1$ représente un halogène, un groupe nitro, alkyle en C1-C4, halogénoalkyle en C1-C4, $-OR_8$, $-OR_9$, hydroxy, cyano ou $-COOR_7$;
$R_5$ représente l'hydrogène, un halogène, un groupe nitro, alkyle en C1-C4, halogénoalkyle en C1-C4, $-OR_8$, $-OR_9$, hydroxy, cyano ou $-COOR_7$; et
X, Y et Z représentent chacun, indépendamment les uns des autres, l'hydrogène, un groupe alkyle en C1-C4, halogénoalkyle en C1-C4, un groupe alkyle en C1-C4 portant un substituant $-OR_9$, un groupe alkyle en C1-C4 portant un substituant $-OR_8$, un halogène, un groupe cycloalkyle en C3-C6 ou un groupe cycloalkyle en C3-C6 substitué par des halogènes, des groupes $-OR_8$, $-OR_9$, $-SR_8$, $-SR_9$ ou alkyle en C1-C4.

**3.** Composés de formule I selon revendication 1, caractérisés en ce que
$R_1$ représente un halogène, un groupe nitro, alkyle en C1-C2, halogénoalkyle en C1-C2, $-OR_8$, $-OR_9$, hydroxy, cyano ou $-COOR_7$;
$R_2$ et $R_4$ représentent chacun, indépendamment l'un de l'autre, l'hydrogène, un halogène, un groupe alkyle en C1-C2, $-COOR_7$ ou $-OR_9$;
$R_5$ représente l'hydrogène, un halogène, un groupe nitro, alkyle en C1-C2, halogénoalkyle en C1-C2, $-OR_8$, $-OR_9$, hydroxy, cyano, $-COOR_7$;
$R_7$ représente un groupe alkyle en C1-C2, alcényle en C2-C3, alcynyle en C2-C3, phényle ou benzyle;
$R_8$ représente un groupe halogénoalkyle en C1-C2;
$R_9$ représente un groupe alkyle en C1-C2; et
X, Y et Z représentent chacun, indépendamment les uns des autres, l'hydrogène, un groupe alkyle en

C1-C2, halogénoalkyle en C1-C2, un halogène, un groupe alkyle en C1-C4 portant un substituant $-OR_9$, un groupe alkyle en C1-C4 portant un substituant $-OR_8$, un groupe cycloalkyle en C3-C6 ou un groupe cycloalkyle en C3-C6 substitué par des halogènes, des groupes $-OR_8$, $-OR_9$ ou alkyle en C1-C2.

**4.** Composés de formule I selon revendication 3, caractérisés en ce que
$R_1$ représente le fluor, le chlore, un groupe méthyle, trifluorométhyle ou méthoxy;
$R_2$ et $R_4$ représentent chacun, indépendamment l'un de l'autre, l'hydrogène, le fluor, le chlore, un groupe méthyle ou méthoxy;
$R_3$ représente l'hydrogène;
$R_5$ représente l'hydrogène, le fluor, le chlore, un groupe méthyle, trifluorométhyle ou méthoxy; et
X, Y et Z représentent chacun, indépendamment les uns des autres, l'hydrogène, un groupe méthyle, cyclopropyle, 2-fluorocyclopropyle, 2,3-difluorocyclopropyle, 2-chlorocyclopropyle, 2,3-dichlorocyclopropyle, 1-méthylcyclopropyle, 2-méthylcyclopropyle, 2,3-diméthylcyclopropyle, cyclopentyle, le fluor, le chlore, un groupe trifluorométhyle ou méthoxy.

**5.** Composés de formule Ia

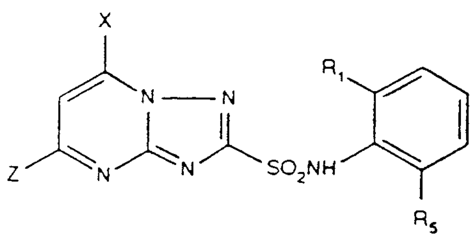

(Ia)

dans laquelle
$R_1$ représente un halogène, un groupe phényle, O-phényle, alkyle en C1-C4, halogénoalkyle en C1-C4, $-OR_8$, $-OR_9$, nitro, hydroxy, cyano, $-SR_6$, $-SOR_6$, $-SO_2R_6$, $-SR_8$, $-SOR_8$, $-SO_2R_8$, $-SO_3R_8$, $-SR_9$, $SOR_9$, $-SO_2R_9$, $-SO_2NR_{10}(R_{10})$, $-SO_3R_9$, $-COOR_7$, $-CONHR_9$, $-CONR_9(R_9)$, formyle,

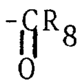, 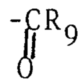,

$-NH_2$, $NHR_9$ ou $-NR_9(R_9)$;
$R_5$ représente l'hydrogène, un halogène, un groupe phényle, O-phényle, alkyle en C1-C4, halogénoalkyle en C1-C4, $-OR_8$, $-OR_9$, nitro, hydroxy, cyano, $-SR_6$, $-SOR_6$, $-SO_2R_6$, $-SR_8$, $-SOR_8$, $-SO_3R_8$, $-SR_9$, $SOR_9$, $-SO_2R_9$, $-SO_2NR_{10}(R_{10})$, $-SO_3R_9$, $-COOR_7$, $-CONHR_9$, $-CONR_9(R_9)$,formyle,

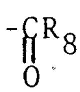, 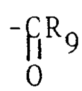,

$-NH_2$, $NHR_9$ ou $-NR_9(R_9)$;
$R_6$ représente l'hydrogène, un groupe phényle, benzyle. alcényle en C2-C4, alcynyle en C2-C4 ou un groupe alkyle en C1-C4 portant un substituant $-OR_9$;
$R_7$ représente un groupe alkyle en C1-C4, alcényle en C2-C4, alcynyle en C2-C4, phényle ou benzyle;
$R_8$ représente un groupe halogénoalkyle en C1-C4;
$R_9$ représente un groupe alkyle en C1-C4;
$R_{10}$ représente l'hydrogène ou un groupe alkyle en C1-C4;
X et Z représentent chacun, indépendamment l'un de l'autre, l'hydrogène, un groupe alkyle en C1-C4, halogénoalkyle en C1-C4, phénylthio, benzylthio, hydroxy, un halogène, un groupe $-OR_8$, $-OR_9$, un groupe alkyle en C1-C4 portant un substituant $-OR_9$, un groupe alkyle en C1-C4 portant un substituant $-OR_8$, un groupe $-NH_2$, $-NHR_9$, $-NR_9(R_9)$, cycloalkyle en C3-C6 ou cycloalkyle en C3-C6 substitué par des halogènes, des groupes $-OR_8$, $-OR_9$, $-SR_8$, $-SR_9$ ou alkyle en C1-C4,
sous réserve que l'un au moins des symboles X et Z représente un groupe cycloalkyle en C3-C6 ou un

groupe cycloalkyle en C3-C6 substitué par des halogènes, des groupes $-OR_8$, $-OR_9$, $-SR_8$, $-SR_9$ ou alkyle en C1-C4.

6. Composés de formule Ia selon revendication 5, caractérisés en ce que
$R_1$ représente un halogène, un groupe nitro, alkyle en C1-C4, halogénoalkyle en C1-C4, $-OR_8$, $-OR_9$, hydroxy, cyano ou $-COOR_7$;
$R_5$ représente l'hydrogène, un halogène, un groupe nitro, alkyle en C1-C4, halogénoalkyle en C1-C4, $-OR_8$, $-OR_9$, hydroxy, cyano ou $-COOR_7$; et
X et Z représentent chacun, indépendamment l'un de l'autre, l'hydrogène, un groupe alkyle en C1-C4, halogénoalkyle en C1-C4, un groupe alkyle en C1-C4 portant un substituant $-OR_9$, un groupe alkyle en C1-C4 portant un substituant $-OR_8$, un halogène, un groupe cycloalkyle en C3-C6 ou un groupe cycloalkyle en C3-C6 substitué par des halogènes, des groupes $-OR_8$, $-OR_9$, $-SR_8$, $-SR_9$ ou alkyle en C1-C4.

7. Composés de formule Ia selon revendication 5, caractérisés en ce que
$R_1$ représente un halogène, un groupe nitro, alkyle en C1-C2, halogénoalkyle en C1-C2, $-OR_8$, $-OR_9$, hydroxy, cyano ou $-COOR_7$,
$R_5$ représente l'hydrogène, un halogène, un groupe nitro, alkyle en C1-C2, halogénoalkyle en C1-C2, $-OR_8$, $-OR_9$, hydroxy, cyano, $-COOR_7$;
$R_7$ représente un groupe alkyle en C1-C2, alcényle en C2-C3, alcynyle en C2-C3, phényle ou benzyle;
$R_8$ représente un groupe halogénoalkyle en C1-C2;
$R_9$ représente un groupe alkyle en C1-C2; et
X et Z représentent chacun, indépendamment l'un de l'autre, l'hydrogène, un groupe alkyle en C1-C2, halogénoalkyle en C1-C2, un halogène, un groupe cycloalkyle en C3-C6 ou un groupe cycloalkyle en C3-C6 substitué par des halogènes, des groupes $-OR_8$, $-OR_9$ ou alkyle en C1-C2.

8. Composés de formule Ia selon revendication 5, caractérisés en ce que
$R_1$ représente le fluor, le chlore, un groupe méthyle, trifluorométhyle ou méthoxy;
$R_5$ représente l'hydrogène, le fluor, le chlore, un groupe méthyle, trifluorométhyle ou méthoxy; et
X et Z représentent chacun, indépendamment l'un de l'autre, l'hydrogène, un groupe méthyle, cyclopropyle, 1-méthylcyclopropyle, 2-fluorocyclopropyle, 2,3-difluorocyclopropyle, 2-chlorocyclopropyle, 2,3-dichlorocyclopropyle, 2-méthylcyclopropyle, 2,3-diméthylcyclopropyle, cyclopentyle, le fluor, le chlore ou un groupe méthoxy.

9. Composés de formule Ia selon revendication 5, caractérisés en ce que
$R_1$ représente le fluor, le chlore, un groupe méthyle ou trifluorométhyle;
$R_5$ représente l'hydrogène, le chlore, un groupe méthyle ou trifluorométhyle; et
X et Z représentent chacun, indépendamment l'un de l'autre, l'hydrogène, un groupe cyclopropyle, 1-méthylcyclopropyle, 2-fluorocyclopropyle, 2,3-difluorocyclopropyle, 2-chlorocyclopropyle, 2,3-dichlorocyclopropyle, 2-méthylcyclopropyle, 2,3-diméthylcyclopropyle, un groupe méthyle, méthoxy, le fluor ou le chlore.

10. Composés de formule Ia selon revendication 9, caractérisés en ce que X représente un groupe cyclopropyle.

11. Composés de formule I selon revendication 1, caractérisés en ce que X représente un groupe cycloalkyle en C3-C6 ou un groupe cycloalkyle en C3-C6 substitué par des halogènes, des groupes $-OR_8$, $-OR_9$, $-SR_8$ $-SR_9$ ou alkyle en C1-C4.

12. Composés de formule I selon revendication 1, caractérisés en ce que X représente un groupe cyclopropyle, 1-méthylcyclopropyle, 2-fluorocyclopropyle, 2,3-difluorocyclopropyle, 2-chlorocyclopropyle, 2,3-dichlorocyclopropyle, 2-méthylcyclopropyle ou 2,3-diméthylcyclopropyle.

13. Composés de formule I selon revendication 1, caractérisés en ce que X représente un groupe cyclopropyle.

14. Le 5-méthyl-7-cyclopropyl-N-(2,6-dichlorophényl)-1,2,4-triazolo[1,5-a]pyrimidine-2-sulfonamide selon revendication 1.

**15.** Procédé de préparation des composés de formule I selon revendication 1, caractérisé en ce que l'on fait réagir une amine primaire de formule II

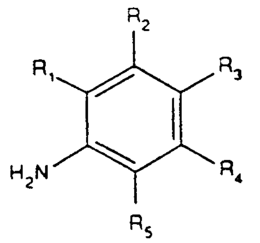

(II)

dans laquelle $R_1$, $R_2$, $R_3$, $R_4$ et $R_5$ ont les significations indiquées en référence à la formule I, en présence d'une base, avec un chlorure de triazolopyrimidinylsulfonyle de formule III

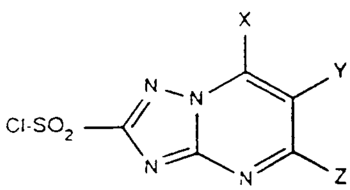

(III)

dans laquelle X, Y et Z ont les significations indiquées en référence à la formule I.

**16.** Un produit herbicide et inhibiteur de la croissance des végétaux, caractérisé en ce qu'il contient, avec des véhicules et/ou d'autres additifs, au moins une substance active consistant en un triazolylsulfonamide de formule I selon revendication 1.

**17.** Produit selon revendication 16, caractérisé en ce qu'il contient de 0,1 à 95% d'une substance active de formule I selon revendication 1.

**18.** Procédé pour combattre les croissances de végétaux indésirables, caractérisé en ce que l'on applique une substance active de formule I selon revendication 1 ou un produit contenant cette substance active, en quantité efficace, sur les végétaux ou leur habitat.

**19.** Procédé selon revendication 18, caractérisé en ce que l'on applique de 0,001 à 5 kg de substance active par hectare.

**20.** Procédé pour inhiber la croissance des végétaux, caractérisé en ce que l'on applique une substance active de formule I selon revendication 1 ou un produit contenant cette substance active, en quantité efficace, sur les végétaux ou leur habitat.

**21.** Procédé pour agir sur la croissance des végétaux en vue d'augmenter les rendements, caractérisé en ce que l'on applique une substance active de formule I selon revendication 1, ou un produit contenant cette substance active, en quantité efficace, sur les végétaux ou leur habitat.

**22.** Procédé selon la revendication 18, pour la lutte sélective en pré-levée ou en post-levée contre les végétaux adventices dans les cultures de végétaux utiles.

**23.** Chlorures de triazolopyrimidinylsulfonyle de formule III

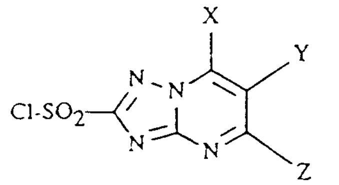

(III),

dans laquelle
X, Y et Z représentent chacun, indépendamment les uns des autres, l'hydrogène, un groupe alkyle en C1-C4, halogénoalkyle en C1-C4, phénylthio, benzylthio, hydroxy, un halogène, un groupe $-OR_8$, $-OR_9$, alkyle en C1-C4 portant un substituant $-OR_9$, alkyle en C1-C4 portant un substituant $-OR_8$, $-NH_2$, $-NHR_9$, $-NR_9(R_9)$, cycloalkyle en C3-C6, cycloalkyle en C3-C6 substitué par des halogènes, des groupes $-OR_8$, $-OR_9$, $-SR_8$, $-SR_9$ ou alkyle en C1-C4, ou bien X et Y, ensemble, ou Y et Z, ensemble, forment un pont alkylène en C2-C3 qui peut être interrompu par l'oxygène ou le soufre, sous que (1) au moins un des symboles X, Y et Z représente un groupe cycloalkyle en C3-C6 ou cycloalkyle en C3-C6 substitué par des halogènes, des groupes $-OR_8$, $-OR_9$, $-SR_8$, $-SR_9$ ou alkyle en C1-C4 et (2) Y ne peut représenter l'hydrogène lorsque X et Z représentent chacun, indépendamment l'un de l'autre, l'hydrogène, un groupe alkyle en C1-C3, trifluorométhyle ou cyclopropyle.

**24.** Composés de formule V

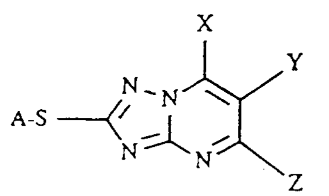

(V),

dans laquelle
X, Y et Z représentent chacun, indépendamment les uns des autres, l'hydrogène, un groupe alkyle en C1-C4, halogénoalkyle en C1-C4, phénylthio, benzylthio, hydroxy, un halogène, un groupe $-OR_8$, $-OR_9$, alkyle en C1-C4 portant un substituant $-OR_9$, alkyle en C1-C4 portant un substituant $-OR_8$, $-NH_2$, $-NHR_9$, $-NR_9(R_9)$, cycloalkyle en C3-C6, cycloalkyle en C3-C6 substitué par des halogènes, des groupes $-OR_8$, $-OR_9$, $-SR_8$, $-SR_9$ ou alkyle en C1-C4, ou bien X et Y, ensemble, ou Y et Z, ensemble, forment un pont alkylène en C2-C3 qui peut être interrompu par l'oxygène ou le soufre; et A représente l'hydrogène, un groupe isopropyle ou benzyle, sous réserve que (1) au moins un des symboles X, Y et Z représente un groupe cycloalkyle en C3-C6 ou cycloalkyle en C3-C6 substitué par des halogènes, des groupes $-OR_8$, $-OR_9$, $-SR_8$, $-SR_9$ ou alkyle en C1-C4, et (2) Y ne peut représenter l'hydrogène lorsque X et Z représentent chacun, indépendamment l'un de l'autre, l'hydrogène, un groupe alkyle en C1-C3, trifluorométhyle ou cyclopropyle.